(19) Europäisches Patentamt / European Patent Office / Office européen des brevets

(11) **EP 4 413 884 A1**

(12) **EUROPEAN PATENT APPLICATION**
published in accordance with Art. 153(4) EPC

(43) Date of publication:
**14.08.2024 Bulletin 2024/33**

(21) Application number: **22878611.7**

(22) Date of filing: **07.10.2022**

(51) International Patent Classification (IPC):
**A24F 40/50** (2020.01)        **A24F 40/51** (2020.01)
**G06F 3/01** (2006.01)

(52) Cooperative Patent Classification (CPC):
**A24F 40/50; A24F 40/51; G06F 3/01**

(86) International application number:
**PCT/JP2022/037557**

(87) International publication number:
**WO 2023/058744 (13.04.2023 Gazette 2023/15)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **08.10.2021 JP 2021166385**

(71) Applicant: **Japan Tobacco, Inc.
Tokyo, 105-6927 (JP)**

(72) Inventors:
• **SHIRAISHI, Takuya
Tokyo 130-8603 (JP)**
• **GOTO, Ayumi
Tokyo 130-8603 (JP)**

(74) Representative: **Hoffmann Eitle
Patent- und Rechtsanwälte PartmbB
Arabellastraße 30
81925 München (DE)**

(54) **FRAGRANCE INHALER OR AEROSOL GENERATING DEVICE, CONTROL METHOD THEREOF, AND PROGRAM THEREOF**

(57)    Provided is a fragrance inhaler or the like controlled on the basis of movement of the fragrance inhaler or the like. A fragrance inhaler or the like (2000) comprises: at least one sense stimulating element (2010) configured to provide a user with sense stimulation; a sensor (2020) configured to detect movement of the fragrance inhaler or the like (2000); and a control unit (2040) configured to cause the at least one sense stimulating element (2010) to function when the sensor (2020) is acquiring input data representing the detected movement.

Fig. 20

**Description**

TECHNICAL FIELD

The present application relates to a flavor inhaler or an aerosol generating device (hereinafter, referred to as "flavor inhaler or the like"). More specifically, the present application relates to a flavor inhaler or the like that is controlled in accordance with a motion of the flavor inhaler or the like.

A flavor inhaler means an apparatus for inhaling a flavor. Although not limited, examples of the flavor inhaler include an electronic tobacco, a heated tobacco, and existing tobaccos. The "aerosol generating device" means a device for inhaling a generated aerosol. Although not limited, examples of the "aerosol generating device" include an electronic tobacco, a heated tobacco, and a medical nebulizer. The flavor inhaler or the like includes so-called reduced-risk products (RRP).

BACKGROUND ART

A flavor inhaler, such as a heated tobacco, is different from a cigarette. A flavor inhaler is often equipped with an electronic apparatus and is proceeding with multifunctionality in recent years. With multifunctionality, a flavor inhaler equipped with a motion sensor has been developed to detect a drop of the flavor inhaler and to detect a specific motion (motion) of the flavor inhaler by a user.

CITATION LIST

PATENT LITERATURE

PTL 1: Japanese Unexamined Patent Application No. 2021-058212
PTL 2: Japanese Unexamined Patent Application Publication (Translation of PCT Application) No. 2017-509339
PTL 3: International Publication No. 2020/008028
PTL 4: International Publication No. 2020/234053

SUMMARY OF INVENTION

TECHNICAL PROBLEM

However, the motion sensor in the flavor inhaler is just used to detect a drop of the flavor inhaler or detect a specific user operation preset to turn on or off a specific function like unlocking the flavor inhaler. It is desired to provide a user with various values of a flavor inhaler by controlling functions of the flavor inhaler in accordance with motion data obtained from the motion sensor.

SOLUTION TO PROBLEM

The present invention is made in view of the above situation, and one of tasks of the present invention is to provide a flavor inhaler or the like that is controlled in accordance with a motion of the flavor inhaler or the like.

An embodiment of the present invention provides a device that is a flavor inhaler or an aerosol generating device. The device includes a vibrator, a sensor configured to detect a motion of the device, a converter configured to convert input data indicating the detected motion to vibration data for vibrating the vibrator, and a controller configured to vibrate the vibrator in accordance with the vibration data.

In one embodiment, the input data may include data indicating an acceleration or angular velocity of the detected motion.

In one embodiment, the converter may be further configured to use a representative value included in the input data.

In one embodiment, using the representative value included in the input data may include converting the representative value to a vibration strength of the vibrator.

In one embodiment, using the representative value included in the input data may include converting the representative value to a vibration pattern of the vibrator.

In one embodiment, the converter may be further configured to divide the input data to a plurality of pieces of data each representing the motion detected in a corresponding one of a plurality of time periods, and convert a representative value included in each of the divided pieces of data to a vibration strength of the vibrator at different timing.

In one embodiment, the sensor may be further configured to detect at least a motion of the device along a first axis and a motion of the device along a second axis, and the input data may include at least first input data representing the detected motion along the first axis and second input data representing the detected motion along the second axis.

In one embodiment, the converter may be further configured to convert the first input data to a vibration strength of the vibrator and convert the second input data to a vibration pattern of the vibrator or select one of a plurality of predetermined vibration patterns as a vibration pattern of the vibrator in accordance with the second input data.

In one embodiment, the vibration pattern may be determined in accordance with at least one of a vibration time, a vibration stop time, and a vibration strength correction coefficient.

In one embodiment, the controller may be further configured to, when inhalation of the device is being performed, vibrate the vibrator in accordance with the vibration data.

In one embodiment, the controller may be further configured to vibrate the vibrator in accordance with the vibration data, and such that a strength of the inhalation and a vibration strength of the vibrator are proportional to each other.

In one embodiment, the controller may be further configured to record an inhalation time that is a length of time during which the inhalation has been being performed, and change, according to the inhalation time, a length of time during which the vibrator is vibrated in accordance with the vibration data.

An embodiment of the present invention provides a control method for a device that is a flavor inhaler or an aerosol generating device and that includes a vibrator. The control method includes a step of detecting a motion of the device, a step of converting input data indicating the detected motion to vibration data for vibrating the vibrator, and a step of vibrating the vibrator in accordance with the vibration data.

An embodiment of the present invention provides a program. The program causes a processor of a device that is a flavor inhaler or an aerosol generating device and that includes a vibrator, to execute a step of detecting a motion of the device, a step of converting input data indicating the detected motion to vibration data for vibrating the vibrator, and a step of vibrating the vibrator in accordance with the vibration data.

An embodiment of the present invention provides a device that is a flavor inhaler or an aerosol generating device. The device includes a sensor configured to detect a motion of the device, a converter configured to convert input data indicating the detected motion to function data for controlling a function of the device, and a controller configured to control the function of the device in accordance with the function data.

In one embodiment, the converter may be further configured to convert continuous values included in the input data to continuous values or a discrete value(s) included in the function data.

In one embodiment, the function of the device may include one or more of a function of heating to generate a flavor, a function of emitting sound, a function of emitting light, and a function of performing predetermined display.

In one embodiment, the input data may include data indicating an acceleration or angular velocity of the detected motion.

In one embodiment, the converter may be further configured to use a representative value included in the input data.

In one embodiment, using a representative value included in the input data may be further configured to convert the representative value to a strength associated with the function of the device.

In one embodiment, using a representative value included in the input data may be further configured to convert the representative value to a pattern associated with the function of the device.

In one embodiment, the converter may be further configured to divide the input data to a plurality of pieces of data each representing the motion detected in a corresponding one of a plurality of time periods, and convert a representative value included in each of the divided pieces of data to a strength associated with the function of the device at different timing.

In one embodiment, the sensor may be further configured to detect at least a motion of the device along a first axis and a motion of the device along a second axis, and the input data may include at least first input data representing the detected motion along the first axis and second input data representing the detected motion along the second axis.

In one embodiment, the converter may be further configured to convert the first input data to a strength associated with the function of the device and convert the second input data to a pattern associated with the function of the device or select one of a plurality of predetermined patterns as a pattern associated with the function of the device in accordance with the second input data.

In one embodiment, the pattern associated with the function of the device may be determined in accordance with at least one of a time during which the function is active, a time during which the function is inactive, and a correction coefficient of a strength associated with the function.

The device according to one embodiment may be configured to heat to generate a flavor and acquire the input data when the heating is not performed.

An embodiment of the present invention provides a control method for a device that is a flavor inhaler or an aerosol generating device. The control method includes a step of detecting a motion of the device, a step of converting input data indicating the detected motion to function data for controlling a function of the device, and a step of controlling the function of the device in accordance with the function data.

An embodiment of the present invention provides a program. The program causes a processor of a device that is a flavor inhaler or an aerosol generating device, to execute a step of detecting a motion of the device, a step of converting input data indicating the detected motion to function data for controlling a function of the device, and a step of controlling the function of the device in accordance with the function data.

An embodiment of the present invention provides a device that is a flavor inhaler or an aerosol generating device. The device includes a vibrator, an inertial sensor, a memory that stores vibration data for vibrating the vibrator, the vibration data being generated by converting inertial data acquired by the inertial sensor, and a controller configured to read the vibration data from the memory and vibrate the vibrator in accordance with the vibration data.

In one embodiment, the vibration data may include a value related to a vibration strength or a vibration time.

In one embodiment, the memory may store the inertial data. The device may further include a converter configured to read the inertial data from the memory and convert the inertial data to the vibration data.

In one embodiment, the inertial sensor may be an angular velocity sensor, and the inertial data may include data indicating an angular velocity.

In one embodiment, the inertial sensor may be an angular velocity sensor, and the inertial data may include data indicating an angular velocity. The converter may be configured to, when the angular velocity is lower than or equal to a predetermined minimum value, convert data indicating the angular velocity to vibration data including a predetermined minimum vibration strength or a predetermined minimum vibration time and, when the angular velocity is higher than or equal to a predetermined maximum value, convert data indicating the angular velocity to vibration data including a predetermined maximum vibration strength or a predetermined maximum vibration time.

In one embodiment, the inertial sensor may be an angular velocity sensor, and the inertial data may include data indicating an angular velocity. The converter may be configured to convert data of which the angular velocity is higher than or equal to 10 dps among data indicating the angular velocity, to the vibration data.

In one embodiment, a sampling rate of the angular velocity sensor may be higher than or equal to 1 Hz and lower than or equal to 1 kHz.

In one embodiment, the device may further include a communicator configured to communicate the inertial data and/or the vibration data with an outside source.

It is another task of the present invention to provide an external apparatus that cooperates with a flavor inhaler or the like as described above.

An embodiment of the present invention provides an apparatus. The apparatus includes a communicator configured to receive, from a device that is a flavor inhaler or an aerosol generating device, input data indicating a motion of the device, detected by a sensor in the device, and a converter configured to convert the input data to vibration data for vibrating a vibrator in the device or function data for controlling a function of the device. The communicator is further configured to send the vibration data or the function data to the device.

In one embodiment, the input data may include data indicating an acceleration or angular velocity of the detected motion.

In one embodiment, the converter may be further configured to use a representative value included in the input data.

In one embodiment, using the representative value included in the input data may include converting the representative value to a vibration strength of the vibrator or a strength associated with the function of the device.

In one embodiment, using the representative value included in the input data may include converting the representative value to a vibration pattern of the vibrator or a pattern associated with the function of the device.

In one embodiment, the converter may be further configured to divide the input data to a plurality of pieces of data each indicating the motion detected in a corresponding one of a plurality of time periods, and convert a representative value included in each of the divided pieces of data to a vibration strength of the vibrator or a strength associated with the function of the device at different timing.

In one embodiment, the sensor may be configured to detect at least a motion of the device along a first axis and a motion of the device along a second axis. The input data may include at least first input data representing the detected motion along the first axis and second input data representing the detected motion along the second axis.

In one embodiment, the converter may be further configured to convert the first input data to a vibration strength of the vibrator or a strength associated with the function of the device and convert the second input data to a vibration pattern of the vibrator or a pattern associated with the function of the device or select one of a plurality of predetermined vibration patterns as a vibration pattern of the vibrator or one of a plurality of predetermined patterns as a pattern associated with the function of the device in accordance with the second input data.

In one embodiment, the vibration pattern may be determined in accordance with at least one of a vibration time, a vibration stop time, and a vibration strength correction coefficient. The pattern associated with the function of the device may be determined in accordance with at least one of a time during which the function is active, a time during which the function is inactive, and a correction coefficient of a strength associated with the function.

In one embodiment, the apparatus may further include a charger configured to charge a rechargeable power supply in the device.

An embodiment of the present invention provides a control method for an apparatus configured to communicate with a device that is a flavor inhaler or an aerosol generating device. The method includes a step of receiving, from the device, input data indicating a motion of the device, detected by a sensor in the device, a step of converting the input data to vibration data for vibrating a vibrator in the device or function data for controlling a function of the device, and a step of sending the vibration data or the function data to the device.

An embodiment of the present invention provides a program. The program causes an apparatus configured to communicate with a device that is a flavor inhaler or an aerosol generating device, to execute a step of receiving, from the device, input data indicating a motion of the device, detected by a sensor in the device, a step of converting the input data to vibration data for vibrating a vibrator in the device or function data for controlling a function of the device, and a step of sending the vibration data or the function data to the device.

An embodiment of the present invention provides a device that is a flavor inhaler or an aerosol generating device. The device includes a vibrator, a sensor configured to detect a motion of the device, a communicator configured to send input data indicating the detected motion to an external apparatus and receive, from the external apparatus, vibration data obtained by converting the input data for vibrating the vibrator or function data obtained by converting the input data for controlling a function of the device, and a controller configured to vibrate the vibrator in accordance with the vibration data or control the function of the device in accordance with the function data.

In one embodiment, the controller may be further configured to, when inhalation of the device is being performed, vibrate the vibrator in accordance with the vibration data or control the function of the device in accordance with the function data.

In one embodiment, the controller may be further configured to vibrate the vibrator in accordance with the vibration data such that a strength of the inhalation and a vibration strength of the vibrator are proportional to each other or control the function of the device in accordance with the function data such that a strength of the inhalation and a strength associated with the function of the device are proportional to each other.

In one embodiment, the controller may be further configured to record an inhalation time that is a length of time during which the inhalation has been being performed and change, according to the inhalation time, a length of time during which the vibrator is vibrated in accordance with the vibration data or a length of time during which the function of the device is active in accordance with the function data.

An embodiment of the present invention provides a method of operating a device that is a flavor inhaler or an aerosol generating device, including a vibrator. The method includes a step of detecting a motion of the device, a step of sending input data indicating the detected motion to an external apparatus, a step of receiving, from the external apparatus, vibration data obtained by converting the input data for vibrating the vibrator or function data obtained by converting the input data for controlling a function of the device, and a step of vibrating the vibrator in accordance with the vibration data or controlling the function of the device in accordance with the function data.

An embodiment of the present invention provides a program. The program causes a device that is a flavor inhaler or an aerosol generating device, including a vibrator, to execute a step of detecting a motion of the device, a step of sending input data indicating the detected motion to an external apparatus, a step of receiving, from the external apparatus, vibration data obtained by converting the input data for vibrating the vibrator or function data obtained by converting the input data for controlling a function of the device, and a step of vibrating the vibrator in accordance with the vibration data or controlling the function of the device in accordance with the function data.

An embodiment of the present invention provides a device that is a flavor inhaler or an aerosol generating device. The device includes at least one sensory stimulation element configured to impart a sensory stimulation to a user, a sensor configured to detect a motion of the device, and a controller configured to, when the sensor is acquiring input data indicating the detected motion, activate the at least one sensory stimulation element.

In one embodiment, the at least one element may include at least one of a vibrator, a light-emitting element, and an acoustic element.

In one embodiment, the input data may include data indicating an acceleration or angular velocity of the detected motion.

In one embodiment, the sensor may be further configured to, when an aerosol is not being generated by heating in the device, acquire the input data.

In one embodiment, the device may include two or more sensory stimulation elements configured to impart a sensory stimulation to a user. The controller may be configured to, while the sensor is in operation, further activate the sensory stimulation element different from the at least one sensory stimulation element of the two or more sensory stimulation elements.

In one embodiment, the device may further include a converter configured to convert the input data to sensory stimulation data for activating the at least one sensory stimulation element.

In one embodiment, the converter may be further configured to use a representative value included in the input data.

In one embodiment, using the representative value included in the input data may include converting the representative value to a strength associated with a sensory stimulation of the at least one sensory stimulation element.

In one embodiment, using the representative value included in the input data may include converting the representative value to a pattern associated with a sensory stimulation of the at least one sensory stimulation element.

In one embodiment, the converter may be further configured to divide the input data to a plurality of pieces of data each representing the motion detected in a corresponding one of a plurality of time periods, and convert a representative value included in each of the divided pieces of data to a strength associated with a sensory stimulation of the at least one sensory stimulation element at different timing.

In one embodiment, the sensor may be further configured to detect at least a motion of the device along a first axis and a motion of the device along a second axis. The input data may include at least first input data representing the detected motion along the first axis and second input data representing the detected motion along the second axis.

In one embodiment, the converter may be further configured to convert the first input data to a strength associated with a sensory stimulation of the at least one sensory stimulation element and convert the second input data to a pattern associated with a sensory stimulation of the at least one sensory stimulation element or select at least one of a plurality of predetermined patterns associated with sensory stimulations as a pattern associated with a sensory stimulation of the at least one sensory stimulation element in accordance with the second input data.

In one embodiment, the pattern associated with the sensory stimulation may be determined in accordance with at least one of a time during which the sensory stimulation element is active, a time during which the sensory stimulation element is inactive, and a correction coefficient of a strength associated with the sensory stimulation.

An embodiment of the present invention provides a control method for a device that is a flavor inhaler or an aerosol generating device. The method includes a step of detecting a motion of the device, and a step of, when input data indicating the detected motion is being acquired, activating at least one sensory stimulation element configured to impart a sensory stimulation to a user.

An embodiment of the present invention provides a program. The program causes a device that is a flavor inhaler or an aerosol generating device, to execute a step of detecting a motion of the device, and a step of, when input data indicating the detected motion is being acquired, activating at least one sensory stimulation element configured to impart a sensory stimulation to a user.

It is further another task of the present invention to provide a configuration of a flavor inhaler or the like, capable of further accurately detecting a motion of the flavor inhaler or the like.

An embodiment of the present invention provides a device that is a flavor inhaler or an aerosol generating device. The device includes a casing, a heater that heats a flavor source or an aerosol source, and an inertial sensor that detects a change in angular velocity or acceleration. The inertial sensor is disposed at a location not in contact with the heater, in the casing.

In one embodiment, any one of three coordinate axes orthogonal to one another in the inertial sensor may be disposed parallel to any one of three coordinate axes orthogonal to one another in the casing.

In one embodiment, the casing may have a thick substantially rectangular parallelepiped shape having a substantially rectangular face, and, where the three coordinate axes in the casing are assumed such that a longitudinal direction of the substantially rectangular shape is a Z-axis, a widthwise direction of the substantially rectangular shape is a Y-axis, and a direction orthogonal to the Z-axis and the Y-axis is an X-axis, the casing and the inertial sensor may be disposed such that an X-axis, a Y-axis, and a Z-axis in the inertial sensor are respectively substantially parallel to the X-axis, the Y-axis, and the Z-axis in the casing.

In one embodiment, the casing may have a substantially cylindrical shape and have a button or a light-emitting element on a surface of the casing, and, for the three coordinate axes in the casing, where a longitudinal direction of the substantially cylindrical shape is a Z-axis, a direction perpendicular to the button or the light-emitting element is a Y-axis, and a direction orthogonal to the Z-axis and the Y-axis is an X-axis, the casing and the inertial sensor may be disposed such that an X-axis, a Y-axis, and a Z-axis in the inertial sensor are respectively substantially parallel to the X-axis, the Y-axis, and the Z-axis in the casing.

In one embodiment, a microcontroller may be further provided, and the inertial sensor may be attached to a substrate to which the microcontroller is attached.

In one embodiment, a microcontroller may be further provided, and the inertial sensor may be attached to a substrate different from a substrate to which the microcontroller is attached.

In one embodiment, in the inertial sensor, at least part of a face on an opposite side to a face that is in contact with a substrate to which the inertial sensor is attached may be covered with a heat insulator.

In one embodiment, the device may have a battery, and the inertial sensor may be disposed at a location closer to a user than the battery when the user inhales a material generated by the flavor inhaler or the aerosol generating device.

In one embodiment, the inertial sensor may be an angular velocity sensor.

BRIEF DESCRIPTION OF DRAWINGS

[Fig. 1A] Fig. 1A is a schematic diagram that schematically illustrates a configuration example of a flavor inhaler or the like according to an embodiment of the present invention.
[Fig. 1B] Fig. 1B is a schematic diagram that schematically illustrates a configuration example of a flavor inhaler or the like according to the embodiment of the present invention.
[Fig. 2] Fig. 2 is a schematic diagram that schematically illustrates a simplified configuration example of a flavor inhaler or the like according to the embodiment of the present invention.
[Fig. 3] Fig. 3 is a graph in which values included in example input data are plotted.
[Fig. 4] Fig. 4 is a graph in which values included in example input data are plotted.
[Fig. 5] Fig. 5 is a schematic diagram of a vibration strength.
[Fig. 6A] Fig. 6A is a table that illustrates a plurality of predetermined vibration patterns.
[Fig. 6B] Fig. 6B is a table that illustrates a plurality of predetermined vibration patterns.
[Fig. 7] Fig. 7 is a flowchart of an example process for vibrating a vibrator in accordance with vibration data.
[Fig. 8A] Fig. 8A is a flowchart of an example process for vibrating a vibrator in accordance with vibration data.
[Fig. 8B] Fig. 8B is a flowchart of an example process for vibrating a vibrator in accordance with vibration data.
[Fig. 9] Fig. 9 is a graph in which changes in pressure detected by a pressure sensor are plotted.
[Fig. 10] Fig. 10 is a schematic diagram that illustrates an example vibration mode of a vibrator.
[Fig. 11] Fig. 11 is a schematic diagram that illustrates an example vibration mode of a vibrator.
[Fig. 12A] Fig. 12A is a schematic diagram that illustrates an example vibration mode of a vibrator.
[Fig. 12B] Fig. 12B is a schematic diagram that illustrates an example vibration mode of a vibrator.
[Fig. 13] Fig. 13 is a flowchart of a control method for a flavor inhaler or the like according to the embodiment of the present invention.
[Fig. 14] Fig. 14 is a schematic diagram that schematically illustrates a simplified configuration example of a flavor inhaler or the like according to an embodiment of the present invention.
[Fig. 15] Fig. 15 is a flowchart of an example process for controlling a function entity in accordance with function data.
[Fig. 16A] Fig. 16A is a flowchart of an example process for controlling a function entity in accordance with function data.
[Fig. 16B] Fig. 16B is a flowchart of an example process for controlling a function entity in accordance with function data.
[Fig. 17] Fig. 17 is a flowchart of a control method for a flavor inhaler or the like according to the embodiment of the present invention.
[Fig. 18] Fig. 18 is a schematic diagram that schematically illustrates a simplified configuration example of a flavor inhaler or the like and an external apparatus according to an embodiment of the present invention.
[Fig. 19] Fig. 19 is a sequence diagram that illustrates an operation of a flavor inhaler or the like and an operation of an external apparatus according to the embodiment of the present invention.
[Fig. 20] Fig. 20 is a schematic diagram that schematically illustrates a simplified configuration example of a flavor inhaler or the like according to an embodiment of the present invention.
[Fig. 21] Fig. 21 is a flowchart of a control method for a flavor inhaler or the like according to the embodiment of the present invention.
[Fig. 22] Fig. 22 is a flowchart that illustrates an example of an operation of a flavor inhaler or the like according to the embodiment of the present invention.
[Fig. 23] Fig. 23 is a flowchart that illustrates an example of an operation of a flavor inhaler or the like according to the embodiment of the present invention.
[Fig. 24] Fig. 24 is a diagram that illustrates an example of a hardware configuration of a flavor inhaler or the like according to an embodiment of the present invention.
[Fig. 25] Fig. 25 is a view that illustrates an example of a state where a user holds a flavor inhaler or the like according to the embodiment of the present invention.
[Fig. 26] Fig. 26 is a diagram that illustrates an arrangement example of a sensor in a flavor inhaler or the like according to the embodiment of the present invention.
[Fig. 27] Fig. 27 is a diagram that illustrates an arrangement example of a sensor in a flavor inhaler or the like according to the embodiment of the present invention.
[Fig. 28] Fig. 28 is a diagram that illustrates an arrangement example of a sensor in a flavor inhaler or the like according to the embodiment of the present invention.

[Fig. 29] Fig. 29 is a diagram that illustrates an arrangement example of a sensor in a flavor inhaler or the like according to the embodiment of the present invention.

[Fig. 30] Fig. 30 is a diagram that illustrates an arrangement example of a sensor in a flavor inhaler or the like according to the embodiment of the present invention.

[Fig. 31] Fig. 31 is a diagram that illustrates an example of a hardware configuration of a flavor inhaler or the like according to the embodiment of the present invention.

[Fig. 32] Fig. 32 is a diagram that illustrates an example of a hardware configuration of a flavor inhaler or the like according to the embodiment of the present invention.

## DESCRIPTION OF EMBODIMENTS

### 1 First Embodiment of Present Invention

A flavor inhaler or the like according to a first embodiment of the present invention is an apparatus that generates a material to be inhaled by a user. Hereinafter, the description will be made on the assumption that a material to be generated by the flavor inhaler or the like is an aerosol. Other than the above, a material to be generated by the flavor inhaler or the like may be a gas not an aerosol.

### 1-1 First Configuration Example

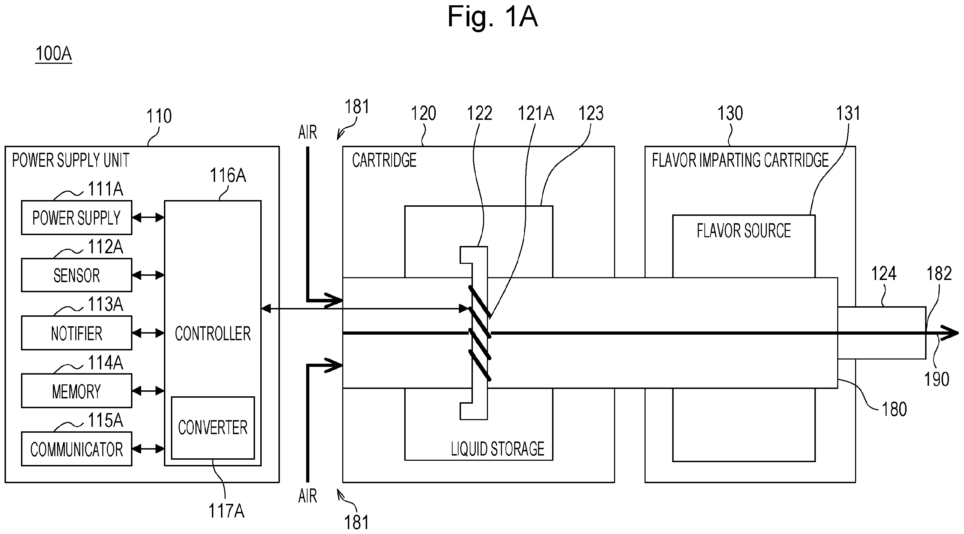

Fig. 1A is a schematic diagram that schematically illustrates a first configuration example of a flavor inhaler or the like. As illustrated in Fig. 1A, the flavor inhaler or the like 100A according to the present configuration example includes a power supply unit 110, a cartridge 120, and a flavor imparting cartridge 130. The power supply unit 110 includes a power supply 111A, a sensor 112A, a notifier 113A, a memory 114A, a communicator 115A, and a controller 116A. The cartridge 120 includes a heater 121A, a liquid guide 122, and a liquid storage 123. The flavor imparting cartridge 130 includes a flavor source 131 and a mouthpiece 124. In the cartridge 120 and the flavor imparting cartridge 130, an airflow path 180 is defined.

The cartridge 120 and the flavor imparting cartridge 130 are examples of a "refill" (described later). In the present embodiment, at least part of one or both of the refills 120, 130 is imparted with a color corresponding to the type of the refill. The one that a color corresponding to a type is imparted is not limited to a refill and may be a selected structural element to be attached to the flavor inhaler or the like 100A.

The power supply 111A stores electric power. The power supply 111A supplies electric power to the structural elements of the flavor inhaler or the like 100A under the control of the controller 116A. The power supply 111A can be a rechargeable battery, such as a lithium ion secondary battery.

The sensor 112A acquires various items of information regarding the flavor inhaler or the like 100A. The sensor 112A may include a pressure sensor such as a microphone capacitor, a flow sensor, or a temperature sensor, and acquire a value generated in accordance with user's inhalation. The sensor 112A may include an input device, such as a button and a switch, that receives input of information from the user. Furthermore, the sensor may include a sensor configured to detect a motion of the flavor inhaler or the like.

The notifier 113A notifies a user of information. The notifier 113A in the present embodiment includes a display device that shows a message. The notifier 113A may include, for example, a light-emitting device or light-emitting element that emits light, a display device that shows an image, a sound output device or an acoustic element that outputs sound, or a vibration device that includes a vibrator, configured to impart a sensory stimulation to a user.

The memory 114A stores various items of information for operation of the flavor inhaler or the like 100A. The memory 114A is, for example, a non-volatile storage medium, such as a flash memory. The memory 114A may include a volatile memory that provides a work area for control with the controller 116A.

The communicator 115A may include a communication interface (including a communication module) that conforms with a predetermined LPWA wireless communication standard or a wireless communication standard having similar limitations. Sigfox, LoRA-WAN, or the like can be adopted as such a communication standard. The communicator 115A is a communication interface capable of communication that conforms with any wired or wireless communication standard. For example, Wi-Fi (registered trademark), Bluetooth (registered trademark), or the like can be adopted as such a communication standard.

The controller 116A functions as an arithmetic processing unit and a control device and controls the overall operations in the flavor inhaler or the like 100A in accordance with various programs. The controller 116A includes an electronic circuit, such as a central processing unit (CPU) and a microprocessor. The controller 116A can include a converter 117A (described in detail later).

The liquid storage 123 stores an aerosol source. The aerosol source is atomized to generate an aerosol. The aerosol source is, for example, a liquid, such as polyhydric alcohol and water. Examples of the polyhydric alcohol include glycerine and propylene glycol. The aerosol source may include a flavor component that is either derived from tobacco or not derived from tobacco. For the flavor inhaler or the like 100A that is a medical inhaler such as a nebulizer, the aerosol source may include a medicine.

The liquid guide 122 guides, from the liquid storage 123, the aerosol source that is the liquid stored in the liquid storage 123, and holds the aerosol source. The liquid guide 122 is, for example, a wick formed by twining fiber material such as glass fiber or porous material such as porous ceramic. In this case, the capillary action of the wick guides the aerosol source stored in the liquid storage 123.

The heater 121A heats the aerosol source to atomize the aerosol source and generate the aerosol. In the example illustrated in Fig. 1A, the heater 121A includes a coil wound around the liquid guide 122. When the heater 121A produces heat, the aerosol source held by the liquid guide 122 is heated and atomized to generate the aerosol. The heater 121A produces heat when receiving electric power from the power supply 111A. In an example, electric power may be supplied in response to the sensor 112A detecting one or both of a start of the user's inhalation and an input of predetermined information. Subsequently, the supply of electric power may be stopped in response to the sensor 112A detecting one or both of an end of the user's inhalation and an input of predetermined information.

The flavor source 131 is a structural element for imparting a flavor component to the aerosol. The flavor source 131 may include a flavor component that is either derived from tobacco or not derived from tobacco.

The airflow path 180 is a flow path of air to be inhaled by a user. The airflow path 180 has a tubular structure having an air inlet hole 181 and an air outlet hole 182 at both ends. The air inlet hole 181 is an inlet of air into the airflow path 180, and the air outlet hole 182 is an outlet of the air from the airflow path 180. The liquid guide 122 is in the airflow path 180 on an upstream side (a side closer to the air inlet hole 181), and the flavor source 131 is in the airflow path 180 on a downstream side (a side closer to the air outlet hole 182). Air flowing in through the air inlet hole 181 as a result of user's inhalation mixes with the aerosol generated by the heater 121A. Subsequently, as indicated by the arrow 190, the mixture fluid of the aerosol and the air passes through the flavor source 131 and is conveyed to the air outlet hole 182. When the mixture fluid of the aerosol and the air passes through the flavor source 131, the flavor component included in the flavor source 131 is imparted to the aerosol.

The mouthpiece 124 is a member to be held in the mouth of a user during inhalation. The mouthpiece 124 has the air outlet hole 182. When a user inhales with the mouthpiece 124 held in his/her mouth, the mixture fluid of the aerosol and the air enters the oral cavity of the user.

The configuration example of the flavor inhaler or the like 100A has been described above. Of course, the flavor inhaler or the like 100A is not limited to the above configuration, and may be configured in various ways as exemplified below.

In an example, the flavor inhaler or the like 100A does not have to include the flavor imparting cartridge 130. In this case, the cartridge 120 includes the mouthpiece 124.

In another example, the flavor inhaler or the like 100A may include a plurality of types of aerosol sources. Still another type of aerosol may be generated by mixing a plurality of types of aerosols generated from the plurality of types of aerosol sources in the airflow path 180 and causing a chemical reaction.

In addition, means for atomizing the aerosol source is not limited to heating by the heater 121A. For example, the means for atomizing the aerosol source may be vibration atomization or induction heating.

1-2 Second Configuration Example

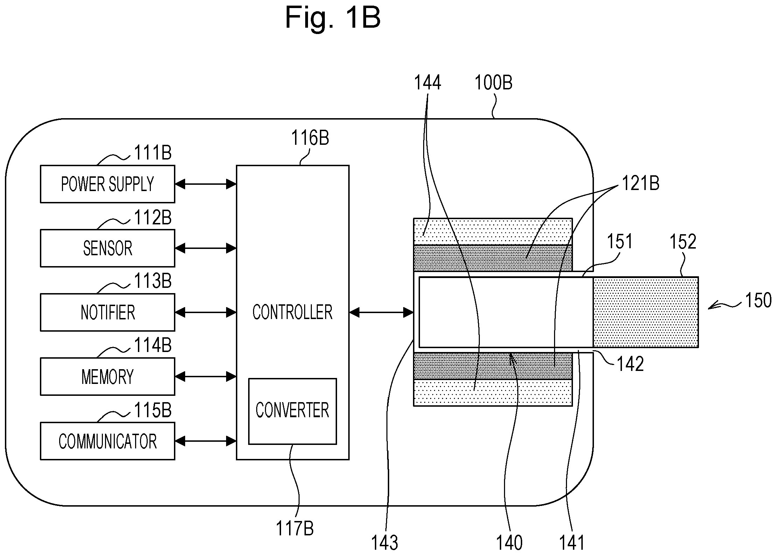

Fig. 1B is a schematic diagram that schematically illustrates a second configuration example of a flavor inhaler or the like. As illustrated in Fig. 1B, the flavor inhaler or the like 100B according to the present configuration example includes a power supply 111B, a sensor 112B, a notifier 113B, a memory 114B, a communicator 115B, a controller 116B, a heater 121B, a holder 140, and a heat insulator 144.

Each of the power supply 111B, the sensor 112B, the notifier 113B, the memory 114B, the communicator 115B, and the controller 116B is substantially the same as a corresponding one of the structural elements included in the flavor inhaler or the like 100A according to the first configuration example.

The holder 140 has an internal space 141. The holder 140 holds the stick substrate 150 while accommodating part of the stick substrate 150 in the internal space 141. The stick substrate 150 is also an example of the "refill". The holder 140 has an opening 142 that allows the internal space 141 to communicate with outside. The holder 140 holds the stick substrate 150 that is inserted into the internal space 141 through the opening 142. For example, the holder 140 is a tubular body having the opening 142 and a bottom 143 at its ends, and defines the pillar-shaped internal space 141. The holder 140 also has the function to define a flow path for air supplied to the stick substrate 150. An air inlet hole that is an inlet for air into the flow path is disposed at, for example, the bottom 143. On the other hand, an air outlet hole that is an outlet for air from the flow path is the opening 142.

The stick substrate 150 includes a substrate 151 and an inhalation port 152. The substrate 151 includes an aerosol source. In the present configuration example, the aerosol source is not limited to a liquid and may be a solid.

The stick substrate 150 held by the holder 140 includes the substrate 151 at least partially accommodated in the internal space 141 and the inhalation port 152 at least partially protruding from the opening 142. When a user inhales with the inhalation port 152 protruding from the opening 142 in his/her mouth, air flows into the internal space 141 through the air inlet hole (not illustrated), and the air and an aerosol generated from the substrate 151 reach the inside of the mouth of the user.

The heater 121B has a similar configuration to that of the heater 121A according to the first configuration example. However, in the example illustrated in Fig.1B, the heater 121B has a film-like shape and surrounds the outer circumference of the holder 140. Subsequently, heat produced from the heater 121B heats the substrate 151 of the stick substrate 150 from the outer circumference, and an aerosol is generated.

The heat insulator 144 prevents heat from transferring from the heater 121B to the other structural elements. For example, the heat insulator 144 may be a vacuum heat insulator or an aerogel heat insulator.

The configuration example of the flavor inhaler or the like 100B has been described above. Of course, the flavor inhaler or the like 100B is not limited to the above configuration, and may be configured in various ways as exemplified below.

In an example, the heater 121B may have a blade-like shape, and may be disposed such that the heater 121B protrudes from the bottom 143 of the holder 140 toward the internal space 141. In this case, the heater 121B having the blade-like shape is inserted into the substrate 151 of the stick substrate 150 and heats the substrate 151 of the stick substrate 150 from its inside. In another example, the heater 121B may be disposed such that the heater 121B covers the bottom 143 of the holder 140. In still another example, the heater 121B may be implemented as a combination of two or more selected from a first heater that covers the outer circumference of the holder 140, a second heater having the blade-like shape, and a third heater that covers the bottom 143 of the holder 140.

In another example, the holder 140 may include an opening/closing mechanism that at least partially opens and closes an outer shell defining the internal space 141. Examples of the opening/closing mechanism include a hinge. In addition, the holder 140 may sandwich the stick substrate 150 inserted into the internal space 141 by opening and closing the outer shell. In this case, the heater 121B may be at the sandwiching position of the holder 140 and may produce heat while pressing the stick substrate 150.

In addition, means for atomizing the aerosol source is not limited to heating by the heater 121B. For example, the means for atomizing the aerosol source may be induction heating.

In addition, the flavor inhaler or the like 100B may further include the heater 121A, the liquid guide 122, the liquid storage 123, and the airflow path 180 according to the first configuration example. The air outlet hole 182 of the airflow path 180 may also serve as an air inlet hole to the internal space 141. In this case, a mixture fluid of the air and an aerosol generated by the heater 121A flows into the internal space 141, mixes further with an aerosol generated by the heater 121B, and then reaches the oral cavity of a user.

1-3 Simplified Configuration Example

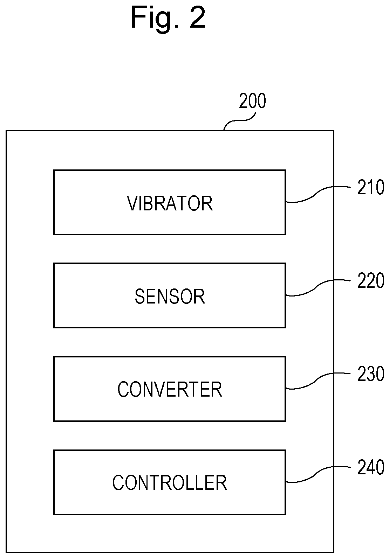

Fig. 2 is a schematic diagram that schematically illustrates a simplified configuration example of only structural elements particularly related to one embodiment of the present invention and extracted from the above-described flavor inhaler or the like 100A or flavor inhaler or the like 100B. Therefore, reference sign 200 indicates the flavor inhaler or the like 100A or the flavor inhaler or the like 100B.

Reference sign 210 indicates the above-described vibrator included in the notifier 113A or the notifier 113B. The vibrator 210 may be regarded as a separate component from the notifier 113A or the notifier 113B. In the following description, it is assumed that vibration of the vibrator 210 is controlled by PWM and the vibration strength is configured to be proportional to a duty ratio of PWM. However, it can be understood that vibration of the vibrator 210 may be controlled by another method.

Reference sign 220 indicates the above-described sensor configured to detect a motion of the flavor inhaler or the like 200 and included in the sensor 112A or the sensor 112B. The sensor may be an inertial sensor (motion sensor), such as an acceleration sensor and an angular velocity sensor (gyro sensor). A sampling rate of the angular velocity sensor may be higher than or equal to 1 Hz and lower than or equal to 1 kHz. Inertial data acquired by the inertial sensor may be stored in the memory 114A or the memory 114B.

Reference sign 230 indicates the above-described converter 117A included in the controller 116A or the above-described converter 117B included in the controller 116B. The converter 230 may be regarded as a separate component from the controller 116A or the controller 116B. The converter 230 is configured to convert data indicating a motion detected by the sensor 220 to data for vibrating the vibrator 210. Here, the former data is regarded as input for outputting the latter data or vibrating the vibrator 210, so, hereinafter, the former data is referred to as "input data". The latter data is data for vibrating the vibrator 210, so, hereinafter, the latter data is referred to as "vibration data". The converter 117A or the converter 117B may read inertial data from the memory 114A or the memory 114B as input data. Vibration data may be stored in the memory 114A or the memory 114B.

Reference sign 240 indicates the controller 116A or the controller 116B. The controller 240 may be regarded as the one excluding the converter 230 from the controller 116A or the controller 116B. The controller 240 is configured to vibrate the vibrator 210 in accordance with vibration data. At this time, the controller 240 may read vibration data from the memory 114A or the memory 114B.

1-4 About Sensor 220 and Input Data

The sensor 220 is configured to repeatedly detect the motion of the flavor inhaler or the like 200. An interval at which the sensor 220 detects the motion of the flavor inhaler or the like 200 may be constant or may be variable. The sensor 220 may be an inertial sensor, such as an acceleration sensor and an angular velocity sensor, as described above, so input data indicating the motion of the flavor inhaler or the like 200 may include multiple sets of the value of acceleration or angular velocity of the motion of the flavor inhaler or the like 200 and time at which the value is detected or an index assigned to the value. Input data may be configured such that a value to which a smaller index is assigned is a value of acceleration or angular velocity of the motion of the flavor inhaler or the like 200, detected earlier. It should be understood that an index may be omitted and does not need to be included in input data. The sensor 220 may be configured to detect only a motion of which the value of acceleration or angular velocity is higher than or equal to a predetermined threshold.

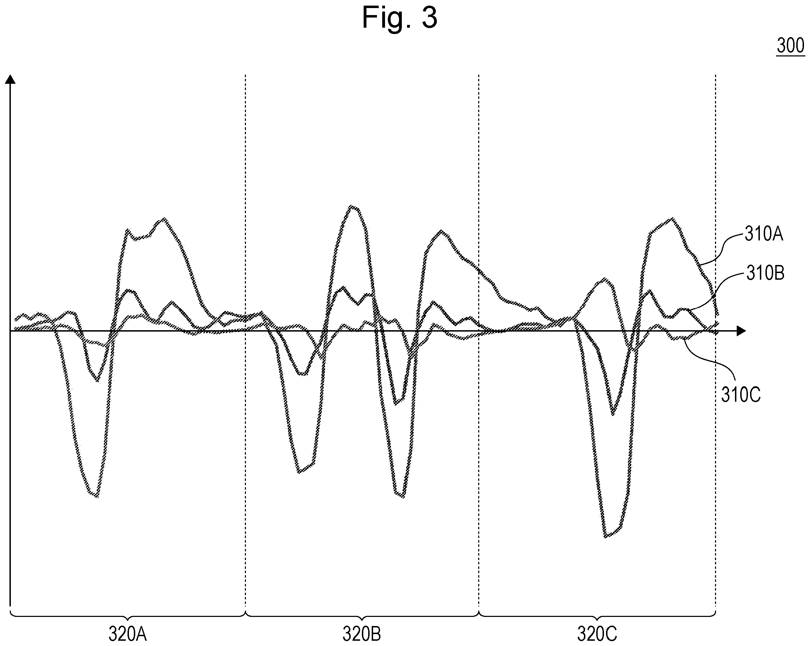

A plurality of axes, generally, three axes orthogonal to one another, are defined for the sensor 220. Therefore, input data may include data indicating the motion of the flavor inhaler or the like 200 on each of the multiple axes, detected by the sensor 220. Hereinafter, data indicating a motion on each axis of the flavor inhaler or the like 200 is referred to as "partial input data". Partial input data may include the value of acceleration or angular velocity of a motion on one axis of the flavor inhaler or the like 200. A motion on an axis means a motion determined with reference to an axis, such as a motion along an axis and a rotating motion around an axis. Therefore, the value of acceleration or angular velocity of a motion on an axis may be the value of acceleration in a direction along the axis or the value of angular velocity in a rotating direction about the axis. Fig. 3 is a graph 300 in which values included in example input data are plotted. The ordinate axis of the graph 300 corresponds to the value of acceleration or angular velocity, and the abscissa axis corresponds to the above-described time or index. Each of reference signs 310A to 310C indicates a plot of values included in each piece of partial input data.

Input data may include only data indicating a motion on a specific one of multiple axes of the flavor inhaler or the like 200. As such a specific axis, for example, an axis on which the value of maximum acceleration or angular velocity is detected by the sensor 220 in a predetermined period may be selected. Alternatively, as such a specific axis, an axis on which the total of the absolute values of accelerations or angular velocities detected by the sensor 220 in a predetermined time period is maximum may be selected.

Input data may include data indicating the one obtained by combining motions on multiple axes of the flavor inhaler or the like 200. Such a combination may be, for example, performed by combining the values of accelerations or angular velocities of motions of the flavor inhaler or the like 200, detected by the sensor 220 at the same time or timing, on the respective ones of the multiple axes.

Input data may include the one obtained by performing a predetermined process on data indicating the motion of the flavor inhaler or the like 200. For example, input data may include a value obtained by smoothing a moving average of the value of acceleration or angular velocity of the motion of the flavor inhaler or the like 200.

Input data includes the value of acceleration or angular velocity of the motion of the flavor inhaler or the like 200, detected by the sensor 220 at different time or timing. Therefore, input data may be divided to a plurality of pieces of data (each includes the value of acceleration or angular velocity of a motion in each time period) each indicating a motion detected in a corresponding one of a plurality of time periods. Each of reference signs 320A to 320C of Fig. 3 indicates a time period corresponding to a corresponding one of the divided pieces of data. The length of time period corresponding to each divided piece of data may be constant or may be different.

1-4 About Converter 230 and Vibration Data

1-5-1 Simple Conversion Approach

Vibration data may include values indicating a plurality of vibration strengths, respectively corresponding to vibrations of the vibrator 210 at different times or timings. Such values D[] can be obtained by the following expression.

$$D[i] = D_{min} + (D_{max} - D_{min}) \times (A[i] - A_{min})/(A_{max} - A_{min}) \qquad (1)$$

Here, $D_{min}$ is a minimum value determined for a vibration strength, $D_{max}$ is a maximum value determined for

a vibration strength, $A_{min}$ is a minimum value determined for input data, $A_{max}$ is a maximum value determined for input data, $A[i]$ is an ith value included in input data, and $D[i]$ is an ith value indicating a vibration strength. i may be regarded as being equivalent to the above-described index. When input data indicates a motion on multiple axes, $A[i]$ may be an ith value included in one of a plurality of pieces of partial input data or may be an ith value of the one obtained by combining a plurality of pieces of partial input data (for example, a total value of ith values respectively included in pieces of partial input data). A minimum value and a maximum value determined on input data may be respectively a minimum value and a maximum value that can be included in input data. $D_{min}$, $D_{max}$, $A_{min}$, and $A_{max}$ may be set to selected values as parameters. It can be understood that the converter 230 that uses the expression (1) can be regarded as being configured to convert $A[]$ that are continuous values included in input data to $D[]$ that are continuous values included in vibration data.

When the input data is less than or equal to the predetermined minimum value ($A[i] \leq A_{min}$), the converter 230 may convert the input data to vibration data including the predetermined minimum vibration strength ($D_{min}$). When the input data is greater than or equal to the predetermined maximum value ($A[i] \geq A_{max}$), the converter 230 may convert the input data to vibration data including the predetermined maximum vibration strength ($D_{max}$).

$A_{max}$ and $A_{min}$ may be set by various methods. For example, $A_{max}$ and $A_{min}$ may be set from the beginning in the flavor inhaler or the like 200. Alternatively, $A_{max}$ and $A_{min}$ may be automatically set by the controller 240 or the like in accordance with the motion of the flavor inhaler or the like 200 resulting from a use of the flavor inhaler or the like 200 by a user. Alternatively, the flavor inhaler or the like 200 may have a mode for setting Amax and Amin. In this case, the flavor inhaler or the like 200 or an external apparatus that communicates with the flavor inhaler or the like 200 may instruct a user to intentionally shake the flavor inhaler or the like 200 strongly or weakly or instruct a user to shake the flavor inhaler or the like 200 for a predetermined time, and the controller 240 may set $A_{max}$ and $A_{min}$ in accordance with the motion of the flavor inhaler or the like 200 at that time.

With such vibration data, it is possible to directly convert a detected motion of the flavor inhaler or the like 200 to vibrations of the vibrator 210 as will be described later.

When the flavor inhaler or the like 200 has a plurality of vibrators, vibration data for each of the vibrators may be converted by using a different piece of partial input data.

1-5-2 Box Approach

The converter 230 may be further configured to use a representative value included in input data. Using a representative value included in input data may be converting a representative value included in input data to a vibration strength or vibration pattern of the vibrator 210.

A representative value included in input data may be a maximum value or extreme value (for example, a local maximum value), a mean, or a median value of values of acceleration or angular velocity of the motion, included in input data (where input data indicates motions on multiple axes, each of a plurality of pieces of partial input data), a value in a predetermined position or in the middle of sequence, included in the data, or the like (hereinafter, referred to as "maximum value or the like"). A representative value may be obtained for each of the above-described divided pieces of data. Therefore, a plurality of representative values can be obtained from input data.

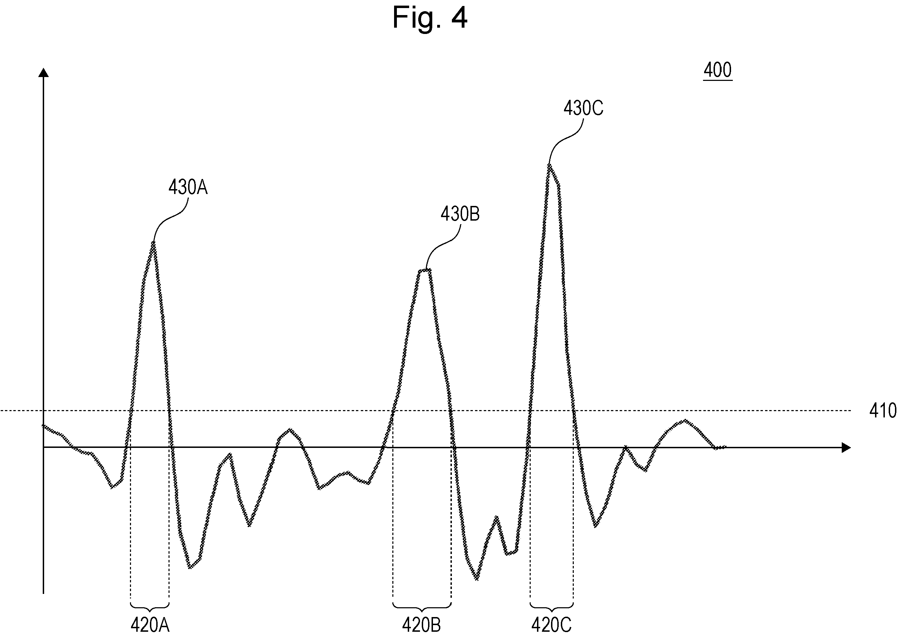

An extreme value may be the one obtained by a method as will be described below. Fig. 4 is a graph 400 in which values included in example input data (which may be regarded as partial input data) are plotted. The ordinate axis of the graph 400 corresponds to the value of acceleration or angular velocity, and the abscissa axis corresponds to the above-described time or index. Reference sign 410 indicates a predetermined threshold, reference signs 420A to 420C indicate portions higher than or equal to the predetermined threshold 410 in the example data. Pieces of data of such portions 420A to 420C of the example data may be respectively used as divided pieces of data, and maximum values 430A to 430C in the respective portions 420A to 420C may be respectively used as extreme values of the divided pieces of data (420A to 420C). When extreme values are obtained by such a method, it should be noted that the number of extreme values obtained changes according to what value the predetermined threshold 410 is set to.

When there is a plurality of maximum values or the like, the maximum values or the like may be sorted in descending order and top predetermined number of the maximum values or the like may be used as representative values.

1-5-2-1 Vibration Strength

Vibration data may include a value indicating a vibration strength that is a reference at the time of vibrating the vibrator 210 (hereinafter, referred to as "reference vibration strength"). Such a value $D_{ref}$ can be obtained by the following expression.

$$D_{ref} = D_{min} + (D_{max} - D_{min}) \times (A_{rep} - A_{min})/(A_{max} - A_{min}) \qquad (2)$$

Here, $A_{rep}$ is a representative value of input data (when input data indicates motions on multiple axes, one of a plurality of pieces of partial input data), and $D_{ref}$ is a value indicating a reference vibration strength. $A_{rep}$ can be obtained from A[] that are continuous values included in input data, so it can be understood that the converter 230 that uses the expression (2) can be regarded as being configured to convert A[] that are continuous values included in input data to $D_{ref}$ that is a discrete value included in vibration data.

When the representative value of input data is less than or equal to the predetermined minimum value ($A_{rep} \leq A_{min}$), the converter 230 may convert the input data to vibration data including the predetermined minimum vibration strength ($D_{min}$). When the representative value of input data is greater than or equal to the predetermined maximum value ($A_{rep} \geq A_{max}$), the converter 230 may convert the input data to vibration data including the predetermined maximum vibration strength ($D_{max}$). As described above, $A_{max}$ and $A_{min}$ may be set by various methods.

Vibration data may include values indicating a plurality of reference vibration strengths, and such values $D_{ref}[]$ can be obtained by the following expression.

$$D_{ref}[j] = D_{min} + (D_{max} - D_{min}) \times (A_{rep}[j] - A_{min})/(A_{max} - A_{min}) \qquad (3)$$

Here, $A_{rep}[j]$ is a jth representative value of input data (when input data indicates motions on multiple axes, one of a plurality of pieces of partial input data), and $D_{ref}[j]$ is a value indicating a jth reference vibration strength. $A_{rep}[j]$ can be obtained from A[] that are continuous values included in input data, so it can be understood that the converter 230 that uses the expression (3) can be regarded as being configured to convert A[] that are continuous values included in input data to $D_{ref}[j]$ that are discrete values included in vibration data.

When the representative value of input data is less than or equal to the predetermined minimum value ($A_{rep}[j] \leq A_{min}$), the converter 230 may convert the input data to vibration data including the predetermined minimum vibration strength ($D_{min}$). When the representative value of input data is greater than or equal to the predetermined maximum value ($A_{rep}[j] \geq A_{max}$), the converter 230 may convert the input data to vibration data including the predetermined maximum vibration strength ($D_{max}$).

1-5-2-2 Vibration Pattern

Vibration data may include a value indicating a vibration pattern at the time of vibrating the vibrator 210. A vibration pattern may be determined by at least one of a vibration time, a vibration stop time, and a vibration strength correction coefficient (described later), and a value indicating a vibration pattern included in vibration data may be at least one of a vibration time, a vibration stop time, a vibration strength correction coefficient, and an index (described later) for selecting one vibration pattern. It should be understood that a vibration pattern may be determined by another parameter in addition to or instead of a vibration time, a vibration stop time, and a vibration strength correction coefficient. For example, a vibration pattern (described later) on an index is determined also by a vibration strength or a reference vibration strength.

1-5-2-2-1 Vibration Time and Vibration Stop Time

The controller 240 is capable of vibrating the vibrator 210 in a mode in which a period during which vibrations are performed and a period during which vibrations are stopped are repeated. For this reason, as described above, a value indicating a vibration pattern may include one or both of a vibration time that is the length of a period during which vibrations are performed and a vibration stop time that is the length of a period during which vibrations are stopped.

Vibration times T[] can be obtained by the following expression.

$$T[k] = T_{min} + (T_{max} - T_{min}) \times (A_{rep}[k] - A_{min})/(A_{max} - A_{min}) \qquad (4)$$

Here, $T_{min}$ is a minimum length determined for a vibration time, $T_{max}$ is a maximum length determined for a vibration time, $A_{rep}[k]$ is a kth representative value of input data (when the input data indicates motions on multiple axes, one of a plurality of pieces of partial input data), and C[k] is a kth vibration strength correction coefficient. $T_{min}$ and $T_{max}$ may be set to selected values as parameters. $A_{rep}[k]$ can be obtained from A[] that are continuous values included in input data, so it can be understood that the converter 230 that uses the expression (4) can be regarded as being configured to convert A[] that are continuous values included in input data to T[k] that are discrete values included in vibration data.

When the representative value of input data is less than or equal to the predetermined minimum value ($A_{rep} \leq A_{min}$), the converter 230 may convert the input data to vibration data including the predetermined minimum vibration

time ($T_{min}$). When the representative value of input data is greater than or equal to the predetermined maximum value ($A_{rep} \geq A_{max}$), the converter 230 may convert the input data to vibration data including the predetermined maximum vibration time ($T_{max}$). As described above, $A_{max}$ and $A_{min}$ may be set by various methods.

Vibration stop times Z[] can be obtained by the following expression.

$$Z[k] = T_0 - T[k] \qquad (5)$$

Here, $T_0$ is the length of one period composed of one period during which vibrations are performed and one period during which vibrations are stopped. $T_0$ may be set to a selected value as a parameter. T[k] can be eventually obtained from A[] that are continuous values included in input data as described above, so it can be understood that the converter 230 that uses the expression (5) can be regarded as being configured to convert A[] that are continuous values included in input data to Z[k] that are discrete values included in vibration data.

Vibration stop times Z[] may be set to fixed values.

1-5-2-2-2 Vibration Strength Correction Coefficient

The controller 240 is capable of deriving one or more vibration strengths from one reference vibration strength. For this reason, as described above, a value indicating a vibration pattern may include a vibration strength correction coefficient for deriving a vibration strength. Vibration strength correction coefficients C[] can be obtained by the following expression.

$$C[k] = C_0 \times (A_{rep}[k] - A_{min})/(A_{max} - A_{min}) \qquad (6)$$

Here, $C_0$ is a predetermined correction coefficient, $A_{rep}[k]$ is a kth representative value of input data (when the input data indicates motions on multiple axes, one of a plurality of pieces of partial input data), and C[k] is a kth vibration strength correction coefficient. The other variables are the same as those of the expression (1). $C_0$ may be set to a selected value as a parameter. $A_{rep}[k]$ can be obtained from A[] that are continuous values included in input data, so it can be understood that the converter 230 that uses the expression (6) can be regarded as being configured to convert A[] that are continuous values included in input data to C[k] that are discrete values included in vibration data.

A method of deriving a vibration strength is selectable, and a value of vibration strength to be derived can be, for example, obtained by multiplying a vibration strength correction coefficient by a value of reference vibration strength. Alternatively, a value $D_{dev}[]$ of vibration strength to be derived can be, for example, obtained by the following expression.

$$D_{dev}[k] = D_{min} + (D_{ref} - D_{min}) \times C[k] \qquad (7)$$

Here, $D_{dev}[k]$ is a kth value of vibration strength to be derived.

Instead of $D_{ref}$, $D_{ref}[k]$ may be used.

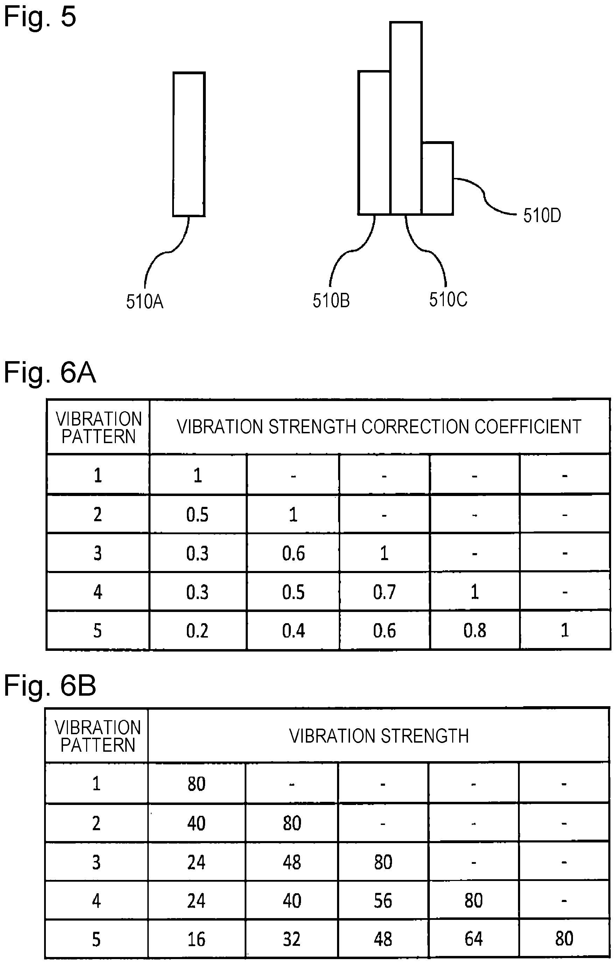

Fig. 5 is a schematic diagram of one reference vibration strength and three derived vibration strengths. Reference sign 510A indicates a schematic block of one reference vibration strength. Reference signs 510B, 510C, and 510D respectively indicate schematic blocks of three vibration strengths derived by multiplying predetermined vibration strength correction coefficients C[1], C[2], C[3] by a value of reference vibration strength 510A. In the diagram, C[1] = 1, C[2] = 1.2, and C[3] = 0.5, and the heights of the blocks 510A to 510D correspond to values of vibration strengths.

A value indicating a vibration pattern may be a vibration time, a vibration stop time, and a vibration strength correction coefficient, associated with each representative value. A vibration pattern PT[k] expressed by a kth representative value can be, for example, expressed as follows.

$$PT[k] = (T[],Z[],C[])$$

When, for example, all the vibration time, vibration stop time, and vibration strength correction coefficient are obtained from the same representative value,

$$PT[k] = (T[k],Z[k],C[k])$$

holds.

In this case, a vibration pattern is present for each representative value. One or some of the vibration time, vibration stop time, and vibration strength correction coefficient may be fixed values.

1-5-2-2-3 Index for Selecting One Vibration Pattern

Alternatively, a value indicating a vibration pattern may be an index for selecting one of a plurality of predetermined vibration patterns. Such a value of index may be equal to the number of representative values included in input data (when the input data indicates motions on multiple axes, one of a plurality of pieces of partial input data). Alternatively, such a value of index may be determined by comparison between a representative value included in input data (when the input data indicates motions on multiple axes, one of a plurality of pieces of partial input data) and a threshold. For example, where the number of predetermined vibration patterns (a maximum value of index) is set to $i_{max}$, the value of index may be one when the representative value is less than a predetermined first threshold, may be two when the representative value is greater than or equal to the predetermined first threshold and less than a second threshold, ... , may be $i_{max}-1$ when the representative value is greater than or equal to a predetermined $(i_{max}-2)$th threshold and less than a predetermined $(i_{max}-1)$th threshold, and may be $i_{max}$ when the representative value is greater than or equal to the predetermined $(i_{max}-1)$ threshold. The number of representative values can be obtained from A[] that are continuous values included in input data, so it can be understood that the converter 230 that uses such a method can be regarded as being configured to convert A[] that are continuous values included in input data to the value of index that is a discrete value included in vibration data.

Each of such a plurality of vibration patterns may be determined by a vibration time, a vibration stop time, and a vibration strength correction coefficient, and one or more of a vibration strength and a reference vibration strength. In other words, a vibration time, a vibration stop time, and a vibration strength correction coefficient, and one or more of a vibration strength and a reference vibration strength, related to vibrations, may be uniquely determined by index.

Figs. 6A and 6B each are a table that illustrates a plurality of predetermined vibration patterns in a case where the number of predetermined vibration patterns (a maximum value of index) is five.

In Fig. 6A, the first vibration pattern is determined by one vibration strength correction coefficient that is 1, the second vibration pattern is determined by two vibration strength correction coefficients that are 0.5 and 1, the third vibration pattern is determined by three vibration strength correction coefficients that are 0.3, 0.5, and 1, the fourth vibration pattern is determined by four vibration strength correction coefficients that are 0.3, 0.5, 0.7, and 1, and the fifth vibration pattern is determined by five vibration strength correction coefficients that are 0.2, 0.4, 0.6, 0.8, and 1.

In Fig. 6B, the first vibration pattern is determined by the value of one vibration strength that is 80, the second vibration pattern is determined by the values of two vibration strengths that are 40 and 80, the third vibration pattern may be determined by the values of three vibration strengths that are 24, 48, and 80, the fourth vibration pattern is determined by the values of four vibration strengths that are 24, 40, 56, and 80, and the fifth vibration pattern is determined by the values of five vibration strengths that are 16, 32, 48, 64, and 80.

1-5-2-3 Others

The converter 230 is capable of obtaining the above-described value indicating a vibration strength and the above-described value indicating a vibration pattern in accordance with different pieces of partial input data. In other words, the converter 230 may convert one (hereinafter, referred to as "first input data") of a plurality of pieces of partial input data to a vibration strength of the vibrator 210 and convert another one (hereinafter, referred to as "second input data") of the plurality of pieces of partial input data to a vibration pattern. Furthermore, second input data and one (hereinafter, referred to as "third input data") of the other pieces of partial input data different from the first input data or the second input data may be converted to a vibration pattern. In other words, for example, a vibration time that determines a vibration pattern may be determined from second input data, and a vibration strength correction coefficient that determines the vibration pattern may be determined from third input data. Alternatively, one of a plurality of predetermined vibration patterns may be selected as a vibration pattern of the vibrator 210 in accordance with second input data.

When the flavor inhaler or the like 200 includes a plurality of vibrators, vibration data for each of the vibrators may be converted by using any one or some of first input data, second input data, and third input data.

The converter 230 may be configured not to use values included in input data and less than a predetermined threshold or values included in input data and not included in a predetermined range, to generate vibration data. When, for example, the sensor 220 is an angular velocity sensor and input data includes data indicating angular velocities, the converter 230 may convert data of which the angular velocity is higher than or equal to 10 dps of the pieces of data indicating angular velocities, to vibration data. Alternatively, the converter 230 may be configured to generate vibration data on the assumption that values included in input data and less than a predetermined threshold or values included in input data and not included in a predetermined range, are predetermined values.

The converter 230 may be configured to generate input data in accordance with output from the sensor 220 to generate vibration data. The converter 230 may be configured to store input data in the memory 114A or the memory 114B and convert the stored input data to vibration data.

The converter 230 may be configured to store the generated vibration data in the memory 114A or the memory 114B. The controller 240 may be configured to use vibration data generated in the past and stored. Vibration data may be editable in the flavor inhaler or the like 200 or by a device external to the flavor inhaler or the like 200.

The converter 230 may be configured to be allowed to select a method of converting input data to vibration data. The controller 240 may be configured to vibrate the vibrator 210 in accordance with vibration data converted by a different method and be allowed to check a vibration mode.

The communicator 115A or the communicator 115B may be configured to communicate inertial data and/or vibration data with an outside source.

1-6 About Controller 240

Hereinafter, a method in which the controller 240 vibrates the vibrator 210 in accordance with vibration data will be described.

1-6-1 Simple Conversion Approach

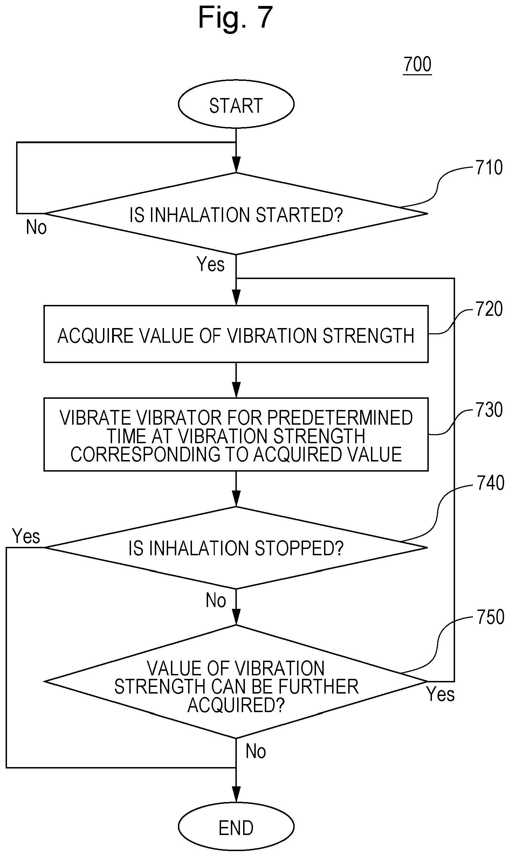

Fig. 7 is a flowchart of an example process 700 for vibrating the vibrator 210 in accordance with vibration data, which is executed by the controller 240.

Reference sign 710 indicates a step of determining whether user's inhalation is started in the flavor inhaler or the like 200. A method of determining whether inhalation is started is selectable. For example, it is possible to perform determination by using a pressure sensor included in the flavor inhaler or the like 200 and configured to detect a pressure change due to inhalation. Particularly, when a pressure strength P (described later) becomes lower than a predetermined threshold, the controller 240 may determine that inhalation is started. When it is determined that inhalation is started, the process proceeds to step 720; otherwise, the process returns to step 710.

Reference sign 720 indicates a step of sequentially acquiring values D[] of vibration strength, included in vibration data.

Reference sign 730 indicates a step of vibrating the vibrator 210 for a predetermined time at the vibration strength corresponding to the acquired value. The predetermined time will be described later.

Reference sign 740 indicates a step of determining whether user's inhalation is stopped in the flavor inhaler or the like 200. A method of determining whether inhalation is stopped is selectable. For example, it is possible to perform determination by using the above-described pressure sensor. Particularly, when the above-described pressure strength P becomes higher than or equal to the predetermined threshold, the controller 240 may determine that inhalation is stopped. When it is determined that inhalation is stopped, the process ends; otherwise, the process proceeds to step 750.

Reference sign 750 indicates a step of determining whether values D[] of vibration strength can be further acquired from vibration data. When not all the values D[] of vibration strength are acquired from vibration data, the controller 240 is allowed to determine that values D[] of vibration strength can be further acquired from vibration data. When it is determined that values D[] of vibration strength can be further acquired from vibration data, the process returns to step 720; otherwise, the process ends.

With the example process 700, it is possible to vibrate the vibrator 210 in accordance with vibration data when inhalation of the flavor inhaler or the like 200 is being performed. With the example process 700, it is possible to directly convert the detected motion of the flavor inhaler or the like 200 to vibrations of the vibrator 210.

A predetermined time in step 730 may be, for example, equal to the length of interval at which the sensor 220 detects the motion of the flavor inhaler or the like 200. In this case, it is possible to temporally synchronize a detected motion of the flavor inhaler or the like 200 with vibrations of the vibrator 210.

Alternatively, a predetermined time in step 730 may be changed with an inhalation time that is the length of time during which inhalation has been being performed in the flavor inhaler or the like 200. For example, a predetermined time in step 730 may be set such that the length of time from when step 720 is executed for the first time to negative determination in step 750 is shorter than the inhalation time, equal to the inhalation time, or longer than the inhalation time. In this case, with an inhalation time, the length of time during which the vibrator 210 is vibrating is compressed or extended. The controller 240 is capable of recording the length of time during which inhalation has been being performed by a user in the past in the flavor inhaler or the like 200 and use such the length of time (or a statistic value, such as a mean of such the length of time) as the above-described inhalation time. The length of time during which inhalation has been being performed by a user in the past in the flavor inhaler or the like 200 may be the length of a period from when the above-described pressure P becomes lower than a predetermined threshold to when the pressure P becomes higher than or equal to the predetermined threshold.

Alternatively, values D[] of vibration strength, included in vibration data acquired in step 720, may be selective. In this case, values D[] of vibration strength included in vibration data may be skipped at predetermined intervals and acquired. When, for example, values D[] of vibration strength included in vibration data are skipped every three values and acquired, values of vibration strength acquired until negative determination is made in step 750 are D[1], D[2], D[4], D[5], D[7], ... (in other words, every three values of vibration strength D[3], D[6], ... are not acquired). A skip interval may be selectively set and may be set according to an inhalation time or an inhalation strength. A skip interval may be variable. For example, a skip interval may be changed according to an inhalation strength. The skip interval may be set to every other values when the inhalation strength is higher than or equal to a first threshold, the skip interval may be set to every three values when the inhalation strength is higher than or equal to a second threshold and lower than the first threshold, ... , the skip interval may be set to every (n+1) values when the inhalation strength is higher than or equal to an nth threshold and lower than an (n-1)th threshold. An interval of the thresholds may be linear or may be selectable. Setting of the skip interval according to an inhalation strength may be performed by measuring an inhalation strength a predetermined number of times during one inhalation or may be performed by measuring an inhalation strength in real time during one inhalation.

1-6-2 Box Approach Part 1

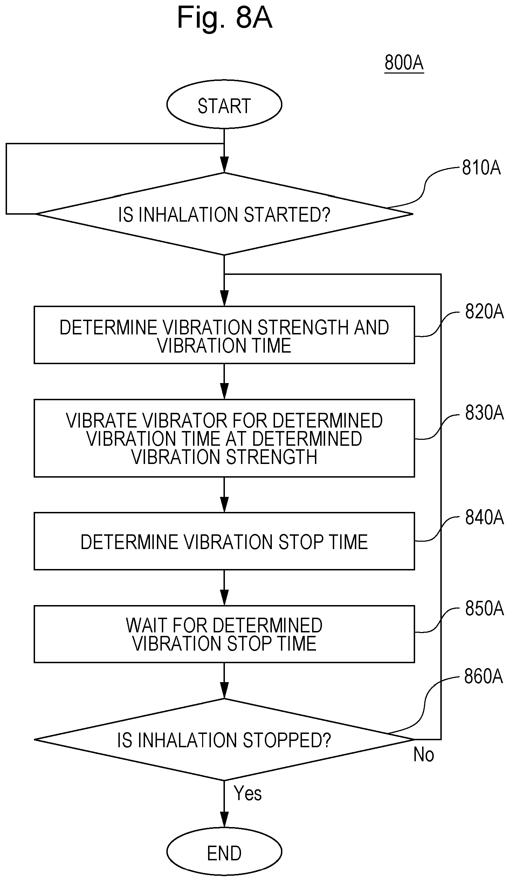

Fig. 8A is a flowchart of an example process 800A for vibrating the vibrator 210 in accordance with vibration data, which is executed by the controller 240.

Reference sign 810A indicates a step of determining whether user's inhalation is started in the flavor inhaler or the like 200. Step 810A may be similar to step 710. When it is determined that inhalation is started, the process proceeds to step 820A; otherwise, the process returns to step 810A.

Reference sign 820A indicates a step of determining a vibration strength and a vibration time at the time of vibrating the vibrator 210. A method of determining a vibration strength and a vibration time will be described later.

Reference sign 830A indicates a step of vibrating the vibrator 210 for the determined vibration time at the determined vibration strength.

Reference sign 840A indicates a step of determining a vibration stop time. A method of determining a vibration stop time will be described later.

Reference sign 850A indicates a step of waiting for the determined vibration stop time.

Reference sign 860A indicates a step of determining whether user's inhalation is stopped in the flavor inhaler or the like 200. Step 860 may be similar to step 740. When it is determined that inhalation is stopped, the process ends; otherwise, the process returns to step 820A.

1-6-2-1 About Determination of Vibration Strength

In step 820A, one of one or more reference vibration strengths of which the values are included in vibration data can be sequentially and cyclically selected as a vibration strength to be determined.

Alternatively, in step 820A, one of one or more vibration strengths derived in accordance with a value of reference vibration strength included in vibration data and one or more vibration strength correction coefficients determined by a vibration pattern can be sequentially and cyclically selected as a vibration strength to be determined.

Alternatively, in step 820A, one of one or more vibration strengths derived in accordance with a value of reference vibration strength included in vibration data and one or more vibration strength correction coefficients of a vibration pattern selected by an index included in vibration data can be sequentially and cyclically selected as a vibration strength to be determined.

Alternatively, in step 820A, one of one or more vibration strengths of a vibration pattern selected by an index included in vibration data can be sequentially and cyclically selected as a vibration strength to be determined.

Alternatively, in step 820A, a vibration strength that is determined in accordance with the above-described vibration data is corrected according to an inhalation strength, and the corrected vibration strength may be adopted as a vibration strength that is finally determined.

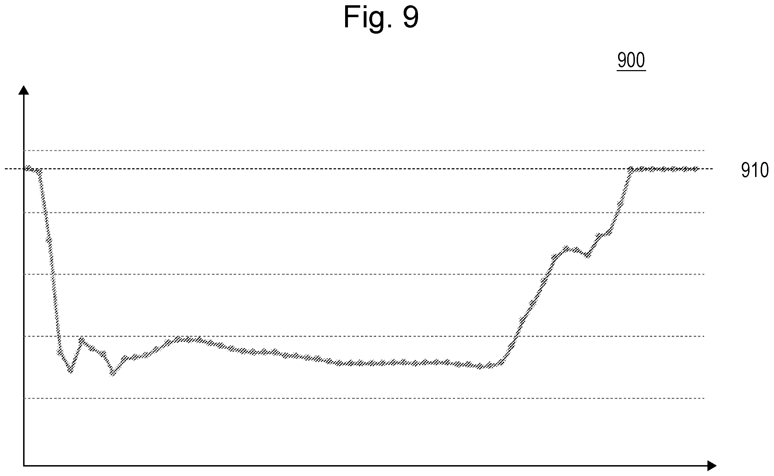

Fig. 9 is a graph 900 in which changes in pressure detected by a pressure sensor are plotted. The ordinate axis of the graph 900 corresponds to a value of detected pressure, and the abscissa axis corresponds to time. Reference sign 910 indicates the value of pressure before inhalation is performed, that is, atmospheric pressure. An inhalation strength P can be obtained by the following expression.

$$P = p_0 - p \qquad (8)$$

Here, $p_0$ is a value of pressure before inhalation is performed, that is, a value of atmospheric pressure, and p is a value of pressure detected at the time when the corresponding step is executed.

A value $D_{detemined}$ of vibration strength that is finally determined in step 820A can be obtained by the following expression.

$$D_{determined} = D_{data} \times P/P_{stn} \qquad (9)$$

Here, $D_{data}$ is a value of vibration strength that is determined in accordance with vibration data as described above, and $P_{stn}$ is a reference inhalation strength. $P_{stn}$ may be a possible maximum inhalation strength $P_{max}$, and $P_{max}$ may be empirically obtained by a selected method, such as inhaling a flavor inhaler or the like as strong as possible. $P_{stn}$ may be a possible ordinary inhalation strength, and such a strength value may be a literature value. $P_{stn}$ may be automatically set by the controller 240 or the like in accordance with inhalation of the flavor inhaler or the like 200 by a user. Alternatively, the flavor inhaler or the like 200 may have a mode for setting $P_{stn}$. In this case, the flavor inhaler or the like 200 or an external apparatus that communicates with the flavor inhaler or the like 200 may instruct a user to inhale the flavor inhaler or the like 200, and the controller 240 may set $P_{stn}$ in accordance with an inhalation strength at that time. With the value $D_{determined}$ of vibration strength obtained in this way, the controller 240 is configured to vibrate the vibrator 210 in accordance with vibration data such that the strength of the inhalation and the vibration strength of the vibrator 210 are proportional to each other.

1-6-2-2 About Determination of Vibration Time

In step 820A, a predetermined length of time may be determined as a vibration time.
Alternatively, in step 820A, a vibration time included in vibration data may be adopted as a vibration time to be determined.

1-6-2-3 About Determination of Vibration Stop Time

In step 840A, a predetermined length of time may be determined as a vibration stop time.
Alternatively, in step 840A, a vibration stop time included in vibration data may be adopted as a vibration stop time to be determined.
Alternatively, in step 840A, a vibration stop time may be determined according to an inhalation strength. Such a vibration stop time Z can be obtained as follows.

$$Z = Z_{max} - (Z_{max} - Z_{min}) \times P/P_{max} \qquad (10)$$

Here, $Z_{min}$ is a minimum value of predetermined vibration stop time, and $Z_{max}$ is a maximum value of predetermined vibration stop time.
Determination of a vibration stop time may be performed together with determination of a vibration time and a vibration strength in step 820A.

1-6-3 Box Approach Part 2

A vibration strength, a vibration time, and a vibration stop time may be determined from vibration data including a reference vibration strength and a vibration pattern and stored in advance. In this case, when inhalation is detected, the controller 240 is capable of reading the one or more different vibration strengths stored, the one or more different vibration times stored, and the one or more different vibration stop times stored and vibrating the vibrator in accordance with the read one or more different vibration strengths stored, the read one or more different vibration times stored, and the read one or more different vibration stop times stored.

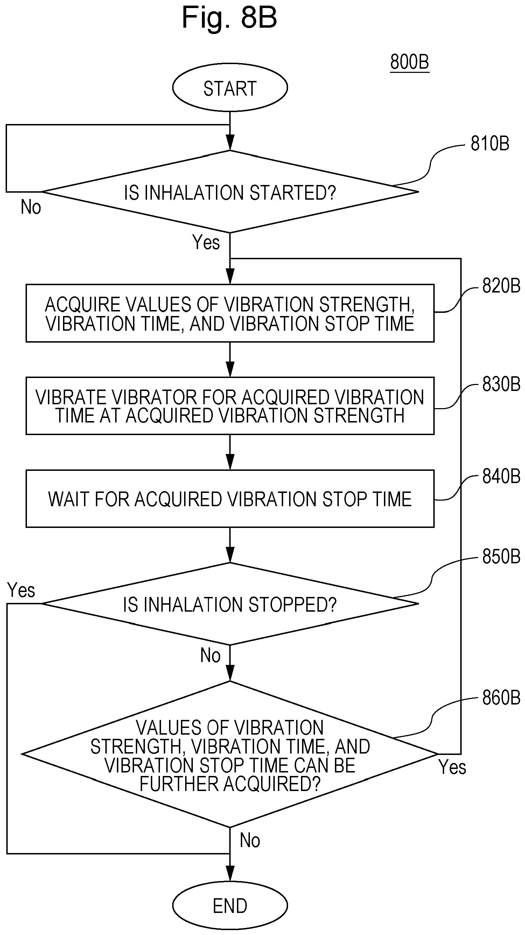

Fig. 8B is a flowchart of another example process 800B for vibrating the vibrator 210 in accordance with vibration data, which is executed by the controller 240.
Reference sign 810B indicates a step of determining whether user's inhalation is started in the flavor inhaler or the like 200. Step 810B may be similar to step 710. When it is determined that inhalation is started, the process proceeds to step 820B; otherwise, the process returns to step 810B.
Reference sign 820B indicates a step of acquiring the values of vibration strength, vibration time, and vibration stop time for vibrating the vibrator 210 from the memory 114A or the memory 114B. A vibration strength, a vibration time, and a vibration stop time to be acquired each may be one of each of one or more different vibration strengths, one of one or more different vibration times, and one of one or more different vibration stop times, determined by the method as described above and stored in the memory 114A or the memory 114B in advance.

Reference sign 830B indicates a step of vibrating the vibrator 210 for the acquired vibration time at the acquired vibration strength.

Reference sign 840B indicates a step of waiting for the acquired vibration stop time.

Reference sign 850B indicates a step of determining whether user's inhalation is stopped in the flavor inhaler or the like 200. Step 850B may be similar to step 740. When it is determined that inhalation is stopped, the process ends; otherwise, the process proceeds to step 860B.

Reference sign 860B indicates a step of determining whether the values of vibration strength, vibration time, and vibration stop time can be further acquired. In step 860B, when there is at least any one of the one or more different vibration strengths, the one or more different vibration times, and the one or more different vibration stop times, stored in the memory 114A or the memory 114B and not yet acquired, it is possible to determine that the values of vibration strength, vibration time, and vibration stop time can be further acquired. When it is determined that the values of vibration strength, vibration time, and vibration stop time can be further acquired, the process returns to step 820B; otherwise, the process ends.

1-6-4 Specific Example of Vibration Mode of Vibrator 210

1-6-4-1 Three Vibration Strengths and Vibration Stop Time Based on Pressure

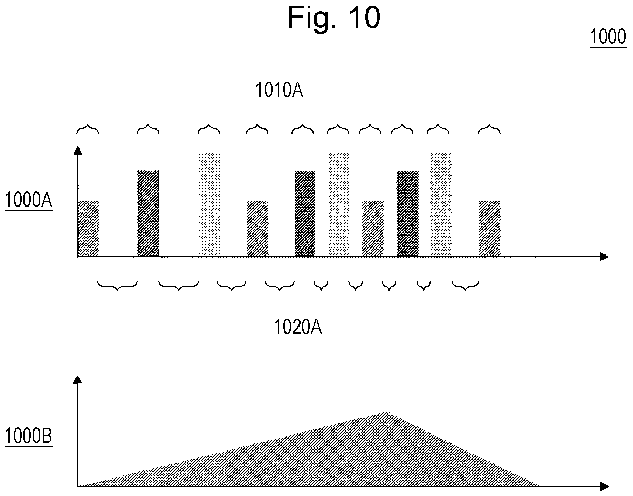

Fig. 10 is a schematic diagram that illustrates an example vibration mode 1000 of the vibrator 210 in the case where three vibration strengths are sequentially and cyclically determined in step 820 and a vibration stop time is determined according to an inhalation strength in step 840.

Reference sign 1000A is a graph that represents a temporal change in the vibration mode of the vibrator 210. The ordinate axis of the graph 1000A corresponds to vibration strength, and the abscissa axis corresponds to time. Therefore, reference sign 1010A indicates each period during which the vibrator 210 is vibrating, and reference sign 1020A indicates each period during which the vibrator 210 is not vibrating. One vibrating period 1010A and one non-vibrating period 1020A on its immediate right correspond to one cycle of execution of step 820 to step 850 of the example process 800. It can be understood from the graph 1000 that vibrations of three different vibration strengths are sequentially repeatedly appearing.

Reference sign 1000B is a graph that represents a change in the inhalation strength of the flavor inhaler or the like 200. The ordinate axis of the graph 1000B corresponds to inhalation strength, and the abscissa axis corresponds to time. The abscissa axis of the graph 1000A and the abscissa axis of the graph 1000B are common.

As is apparent from the graph 1000A and the graph 1000B, it can be understood that the length of non-vibrating period 1020A is changing according to an inhalation strength, more specifically, the length of non-vibrating period 1020A shortens as the inhalation strength increases.

1-6-4-2 One Vibration Strength and Vibration Stop Time Based on Pressure

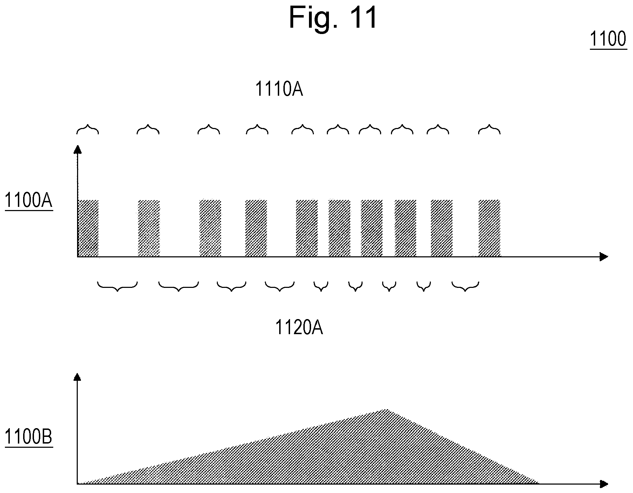

Fig. 11 is a schematic diagram that illustrates an example vibration mode 1100 of the vibrator 210 in the case where one vibration strength is sequentially and cyclically determined in step 820, that is, one vibration strength is continuously determined, and a vibration stop time is determined according to an inhalation strength in step 840.

Reference sign 1100A is a graph that represents a temporal change in the vibration mode of the vibrator 210. The ordinate axis of the graph 1100A corresponds to vibration strength, and the abscissa axis corresponds to time. Therefore, reference sign 1110A indicates each period during which the vibrator 210 is vibrating, and reference sign 1120A indicates each period during which the vibrator 210 is not vibrating. One vibrating period 1110A and one non-vibrating period 1120A on its immediate right correspond to one cycle of execution of step 820 to step 850 of the example process 800. It can be understood from the graph 1100 that only vibrations of one vibration strength are repeatedly appearing.

Reference sign 1100B is a graph that represents a change in the inhalation strength of the flavor inhaler or the like 200. The ordinate axis of the graph 1100B corresponds to inhalation strength, and the abscissa axis corresponds to time. The abscissa axis of the graph 1100A and the abscissa axis of the graph 1100B are common.

As is apparent from the graph 1100A and the graph 1100B, it can be understood that the length of non-vibrating period 1120A is changing according to an inhalation strength, more specifically, the length of non-vibrating period 1120A shortens as the inhalation strength increases.

1-6-4-3 One Vibration Strength and Vibration Strength and Vibration Stop Time Based on Pressure

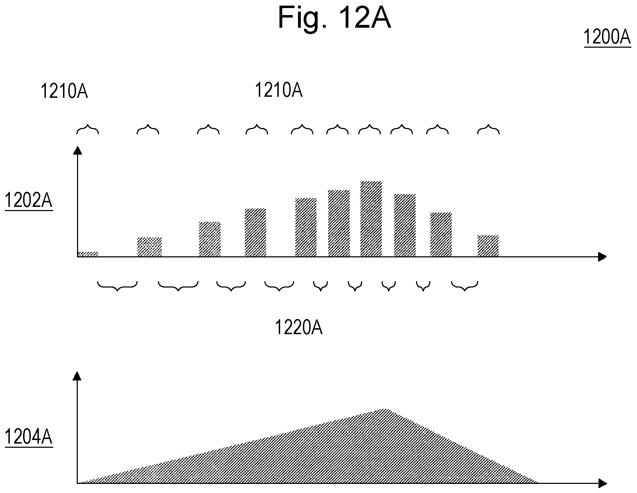

Fig. 12A is a schematic diagram that illustrates an example vibration mode 1200A of the vibrator 210 in the case where one vibration strength is sequentially and cyclically determined in step 820A, that is, one vibration strength is continuously determined, the vibration strength is corrected according to an inhalation strength in step 840A, and a vibration stop time is determined according to an inhalation strength in step 840A.

Reference sign 1202A is a graph that represents a temporal change in the vibration mode of the vibrator 210. The ordinate axis of the graph 1202A corresponds to vibration strength, and the abscissa axis corresponds to time. Therefore, reference sign 1210A indicates each period during which the vibrator 210 is vibrating, and reference sign 1220A indicates each period during which the vibrator 210 is not vibrating. One vibrating period 1210A and one non-vibrating period 1220A on its immediate right correspond to one cycle of execution of step 820A to step 850A of the example process 800A.

Reference sign 1204A is a graph that represents a change in the inhalation strength of the flavor inhaler or the like 200. The ordinate axis of the graph 1204A corresponds to inhalation strength, and the abscissa axis corresponds to time. The abscissa axis of the graph 1202A and the abscissa axis of the graph 1204A are common.

It can be understood from the graph 1200A that a vibration strength proportional to an inhalation strength is appearing. As is apparent from the graph 1202A and the graph 1204A, it can be understood that the length of non-vibrating period 1220A is changing according to an inhalation strength, more specifically, the length of non-vibrating period 1220A shortens as the inhalation strength increases.

1-6-4-4 Three Vibration Strengths and Vibration Strength and Vibration Stop Time Based on Pressure

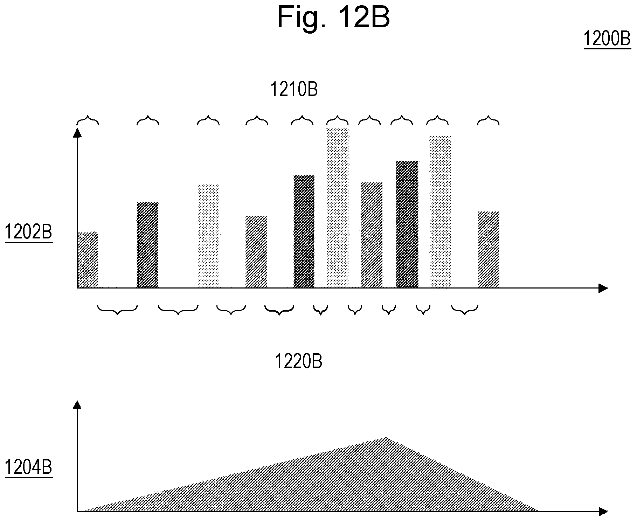

Fig. 12B is a schematic diagram 1200B that illustrates an example vibration mode of the vibrator 210 in the case where three vibration strengths are sequentially and cyclically determined in step 840A, the vibration strength is corrected according to an inhalation strength in step 820A, and a vibration stop time is determined according to an inhalation strength in step 840A.

Reference sign 1202B is a graph that represents a temporal change in the vibration mode of the vibrator 210. The ordinate axis of the graph 1202B corresponds to vibration strength, and the abscissa axis corresponds to time. Therefore, reference sign 1210B indicates each period during which the vibrator 210 is vibrating, and reference sign 1220B indicates each period during which the vibrator 210 is not vibrating. One vibrating period 1210B and one non-vibrating period 1220B on its immediate right correspond to one cycle of execution of step 820A to step 850A of the example process 800A.

Reference sign 1204B is a graph that represents a change in the inhalation strength of the flavor inhaler or the like 200. The ordinate axis of the graph 1204B corresponds to inhalation strength, and the abscissa axis corresponds to time. The abscissa axis of the graph 1202B and the abscissa axis of the graph 1204B are common.

It can be understood from the graph 1200B that a vibration strength proportional to an inhalation strength is appearing. As is apparent from the graph 1202B and the graph 1204B, it can be understood that the length of non-vibrating period 1220B is changing according to an inhalation strength, more specifically, the length of non-vibrating period 1220B shortens as the inhalation strength increases.

1-7 Overall Flow of Control of Flavor Inhaler or the Like 200

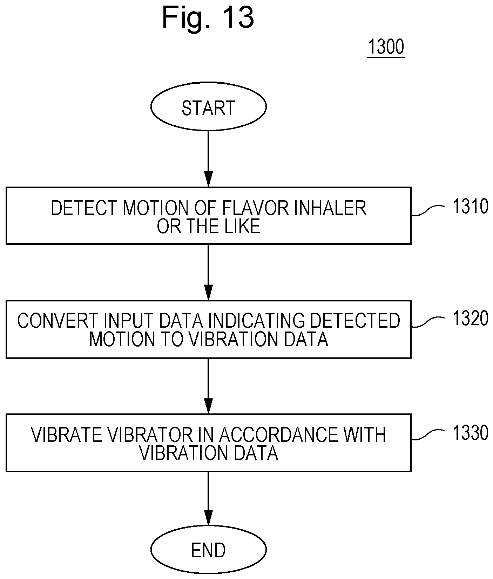

Fig. 13 is a flowchart of a control method 1300 for the flavor inhaler or the like 200.

Reference sign 1310 indicates a step of detecting the motion of the flavor inhaler or the like 200. This step may be the one that the sensor 220 included in the flavor inhaler or the like 200 executes. This step may be the one that the processor included in the flavor inhaler or the like 200 executes by using the sensor 220. A step of detecting the motion of the flavor inhaler or the like 200 may be executed after a user makes a predetermined operation (such as depressing a button) on the flavor inhaler or the like 200 or may be executed at other various timings.

Reference sign 1320 indicates a step of converting input data indicating the detected motion of the flavor inhaler or the like 200 to vibration data for vibrating the vibrator 210 included in the flavor inhaler or the like 200. This step may be the one that the converter 230 included in the flavor inhaler or the like 200 executes. This step may be the one that the processor included in the flavor inhaler or the like 200 executes as the converter 230.

Reference sign 1330 indicates a step of vibrating the vibrator 210 in accordance with the vibration data acquired in step 1320. This step may be the one that the controller 240 included in the flavor inhaler or the like 200 executes and may be the one including the above-described example process 700 or example process 800. This step may be the one that the processor included in the flavor inhaler or the like 200 executes as the controller 240. A step of vibrating the vibrator 210 in accordance with vibration data may be executed during inhalation of the flavor inhaler or the like 200 by a user, or may be executed in response to a predetermined operation (such as depressing a button) on the flavor inhaler or the like 200 by a user, or may be executed at other various timings. After the controller 240 creates vibration data, the controller 240 may automatically vibrate the vibrator 210 in accordance with the vibration data so that a user can check the vibration data.

Of course, a program may cause the processor of the flavor inhaler or the like 200 to execute the control method 1300.

The power supply 111A or power supply 111B of the flavor inhaler or the like 200 may include a rechargeable battery. In this case, the battery may be charged by a charging apparatus electrically connected to the flavor inhaler or the like 200. The charging apparatus may include not only the charger but also at least one of a vibrator, a sensor, a converter, a controller, and the like similar to the structural elements illustrated in Fig. 2. When the flavor inhaler or the like 200 is connected to the charging apparatus as described above, at least some of the steps of the control method 1300 may be executed by the structural elements in the charging apparatus.

According to the first embodiment of the present invention, when a user moves the flavor inhaler or the like, a variety of vibration data is generated. It is possible to vibrate the flavor inhaler or the like in a variety of modes in accordance with the generated vibration data. For this reason, a user is able to inhale the flavor inhaler or the like while the user feels vibrations in a variety of modes including a mode that the user cannot predict. Therefore, it is possible to improve user experience.

2 Second Embodiment of Present Invention

The flavor inhaler or the like according to the first embodiment is configured to vibrate the vibrator in accordance with vibration data converted from input data. On the other hand, a flavor inhaler or the like according to a second embodiment of the present invention is configured to control a predetermined function of the flavor inhaler or the like in accordance with the one corresponding to vibration data converted from input data. Therefore, the flavor inhaler or the like according to the second embodiment of the present invention may be the same as the flavor inhaler or the like according to the first embodiment of the present invention except that it is not necessary to include a vibrator.

Hereinafter, the flavor inhaler or the like according to the second embodiment will be described. In the following description, the one corresponding to vibration data is referred to as "function data", the one corresponding to "vibration strength" is referred to as "function strength", the one corresponding to vibration pattern is referred to as "function pattern", the one corresponding to vibration time is referred to as "function time", the one corresponding to vibration stop time is referred to as "function stop time", and the one corresponding to vibration strength correction coefficient is referred to as "function strength correction coefficient". Therefore, the details and deriving methods of function data, function strength, function pattern, function time, function stop time, and function strength correction coefficient may be respectively the same as those of vibration data, vibration strength, vibration pattern, vibration time, vibration stop time, and vibration strength correction coefficient.

2-1 Simplified Configuration Example

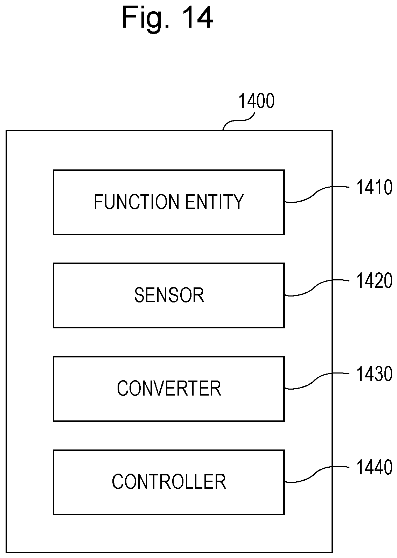

Fig. 14 is a schematic diagram that schematically illustrates a simplified configuration example of only structural elements particularly related to the present embodiment and extracted from the above-described flavor inhaler or the like 100A or flavor inhaler or the like 100B. Therefore, reference sign 1400 indicates the flavor inhaler or the like 100A or the flavor inhaler or the like 100B.

Reference sign 1410 indicates a function entity that is controlled in the present embodiment. The function entity is, for example, the heater 121A or the heater 121B, the light-emitting device or light-emitting element that is included in the notifier 113A or the notifier 113B, the display device that shows an image and that is included in the notifier 113A or the notifier 113B, or the sound output device or acoustic element that outputs sound and that is included in the notifier 113A or the notifier 113B; however, the function entity is not limited thereto.

A function strength of the light-emitting device or light-emitting element may be the one that controls one or both of the intensity of light emission and the color of light emission (capable of allocating a different color according to a function strength). A function strength of the sound output device or acoustic element may be the one that controls at least one of the strength of sound, the pitch of sound, and the type of sound (capable of allocating a different sound according to a function strength). A function strength of the display device may be the one that controls at least one of the hue of display, the lightness of display, the chroma of display, and information displayed (including an image, and different information is displayed according to a function strength). A function strength of the heater 121A or the heater 121B may be the one that controls one or more of the temperature of the heater 121A or the heater 121B, a current flowing through the heater 121A or the heater 121B, a voltage applied to the heater 121A or the heater 121B, and an electric power supplied to the heater 121A or the heater 121B.

Reference sign 1420 indicates the one similar to the sensor 220.

Reference sign 1430 indicates the one similar to the converter 230.

Reference sign 1440 indicates the one similar to the controller 240 except that the controller 1440 does not vibrate a vibrator in accordance with vibration data but controls the function entity 1410 in accordance with function data.

2-2 Simple Conversion Approach

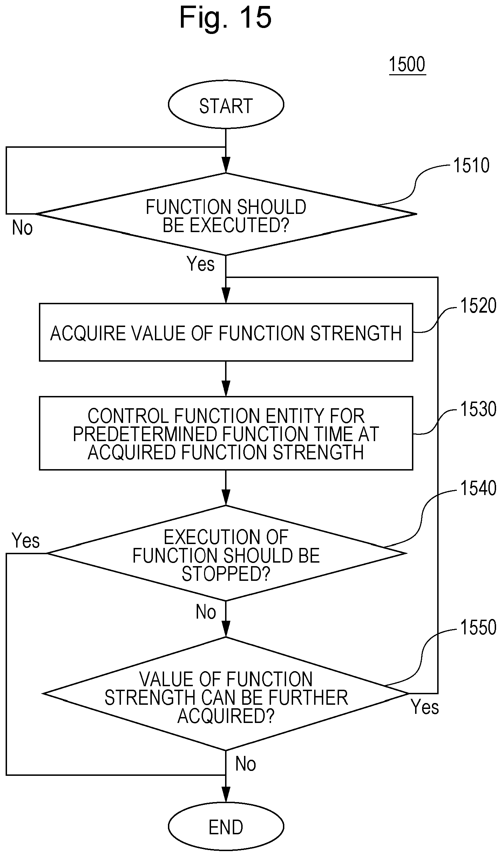

Fig. 15 is a flowchart of an example process 1500 for controlling the function entity 1410 in accordance with function data, which is executed by the controller 1440.

Reference sign 1510 indicates a step of determining whether to execute the function of the function entity 1410 in the flavor inhaler or the like 1400. This determination may be a selected different determination depending on the substance of the function entity 1410. For example, this determination may be a determination as to whether a button or a switch included in the sensor 112A or the sensor 112B is depressed. Alternatively, when the function entity 1410 is the heater 121A or the heater 121B, this determination may be, for example, a determination as to whether inhalation is started. When it is determined to execute the function, the process proceeds to step 1520; otherwise, the process returns to step 1510.

Reference sign 1520 indicates a step of sequentially acquiring values of function strength (which correspond to D[] of vibration strength) included in function data.

Reference sign 1530 indicates a step of controlling the function entity 1410 for a predetermined time at the acquired value of function strength.

Reference sign 1540 indicates a step of determining whether to terminate execution of the function of the function entity 1410 in the flavor inhaler or the like 1400. This determination may be a selected different determination depending on the substance of the function entity 1410. For example, this determination may be a determination as to whether a button or a switch included in the sensor 112A or the sensor 112B is depressed again. Alternatively, when the function entity 1410 is the heater 121A or the heater 121B, this determination may be, for example, a determination as to whether inhalation is stopped. When it is determined that execution of the function should be terminated, the process ends; otherwise, the process proceeds to step 1550.

Reference sign 1550 indicates a step of determining whether a value of function strength can be further acquired from function data. When not all the values of function strength are acquired from function data, the controller 1440 is allowed to determine that a value of function strength can be further acquired from the function data. When it is determined that a value of function strength can be further acquired from function data, the process returns to step 1520; otherwise, the process ends.

With the example process 1500, the detected motion of the flavor inhaler or the like 1400 can be directly incorporated into control over the function entity 1410.

2-3 Box Approach (Part 1)

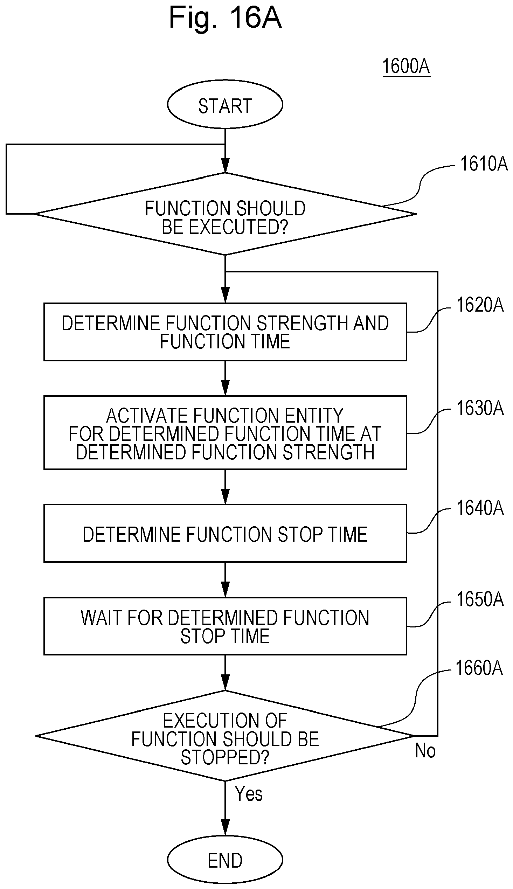

Fig. 16A is a flowchart of an example process 1600A for controlling the function entity 1410 in accordance with function data, which is executed by the controller 1440.

Reference sign 1610A indicates a step of determining whether to execute the function of the function entity 1410 in the flavor inhaler or the like 1400. Step 1610A may be similar to step 1510. When it is determined to execute the function, the process proceeds to step 1620A; otherwise, the process returns to step 1610A.

Reference sign 1620A indicates a step of determining a function strength and a function time for controlling the function entity 1410.

Reference sign 1630A indicates a step of controlling the function entity 1410 for the determined function time at the determined function strength.

Reference sign 1640A indicates a step of determining a function stop time.

Reference sign 1650A indicates a step of waiting for the determined function stop time.

Reference sign 1660A indicates a step of determining whether to terminate execution of the function of the function entity 1410 in the flavor inhaler or the like 1400. Step 1660A may be similar to step 1540. When it is determined that execution of the function should be terminated, the process ends; otherwise, the process returns to step 1620A.

2-4 Box Approach (Part 2)

A function strength, a function time, and a function stop time may be determined from function data including a reference function strength and a function pattern and stored in advance. In this case, when it is determined to execute the function, the controller 240 is capable of reading the one or more different function strengths stored, the one or more different function times stored, and one or more different function stop times stored and controlling the function entity 1410 in accordance with the read one or more different function strengths stored, the read one or more different function times stored, and the read one or more different function stop times stored.

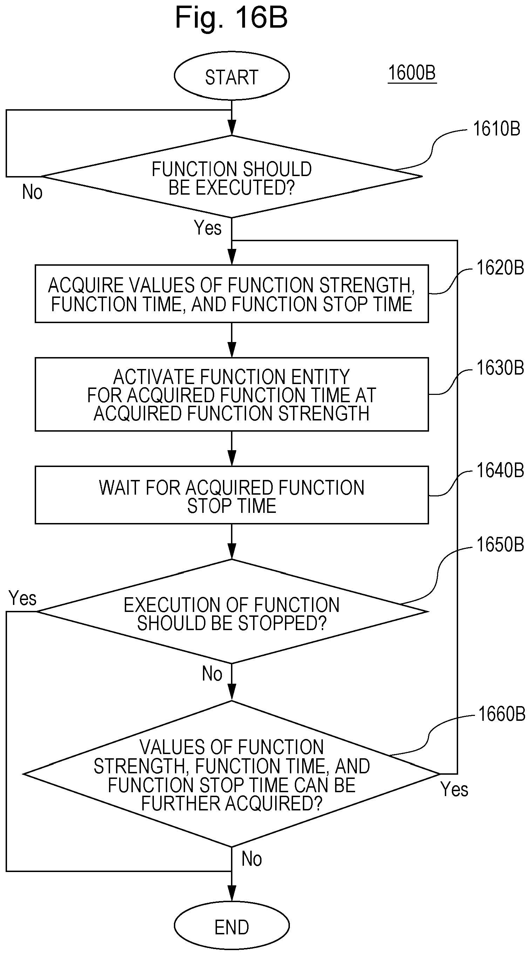

Fig. 16B is a flowchart of an example process 1600B for controlling the function entity 1410 in accordance with function data, which is executed by the controller 1440.

Reference sign 1610B indicates a step of determining whether to execute the function of the function entity 1410 in the flavor inhaler or the like 1400. Step 1610B may be similar to step 1510. When it is determined to execute the function, the process proceeds to step 1620B; otherwise, the process returns to step 1610B.

Reference sign 1620B indicates a step of acquiring the values of function strength, function time, and function stop time for controlling the function entity 1410 from the memory 114A or the memory 114B. A function strength, a function time, and a function stop time to be acquired each may be one of each of one or more different function strengths, one or more different function times, and one or more different function stop times, determined by the method as described above and stored in the memory 114A or the memory 114B in advance.

Reference sign 1630B indicates a step of controlling the function entity 1410 for the acquired function time at the acquired function strength.

Reference sign 1640B indicates a step of waiting for the acquired function stop time.

Reference sign 1650B indicates a step of determining whether to terminate execution of the function of the function entity 1410 in the flavor inhaler or the like 1400. Step 1650B maybe similar to step 1540. When it is determined to terminate execution of the function, the process proceeds to step 1660B; otherwise, the process returns to step 1620B.

Reference sign 1660B indicates a step of determining whether the values of function strength, function time, and function stop time can be further acquired. In step 1660B, when there is at least anyone of the one or more different function strengths, the one or more different function times, and the one or more different function stop times, stored in the memory 114A or the memory 114B and not yet acquired, it is possible to determine that the values of function strength, function time, and function stop time can be further acquired. When it is determined that the values of function strength, function time, and function stop time can be further acquired, the process returns to step 1620B; otherwise, the process ends.

2-5 Overall Flow of Control of Flavor Inhaler or the Like 1400

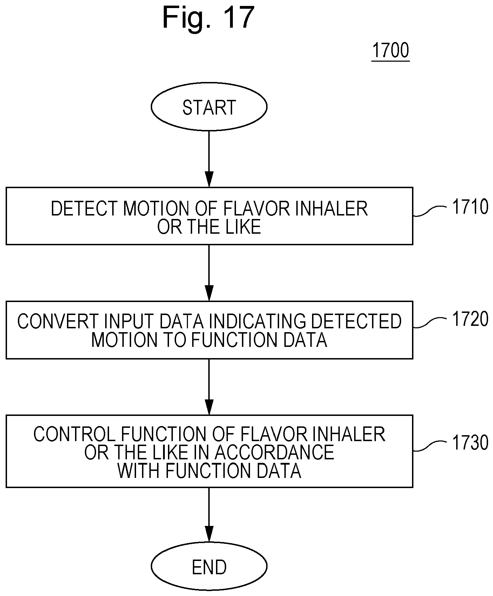

Fig. 17 is a flowchart of a control method 1700 for the flavor inhaler or the like 1400.

Reference sign 1710 indicates a step of detecting the motion of the flavor inhaler or the like 1400. This step may be the one that the sensor 1420 included in the flavor inhaler or the like 1400 executes. This step may be the one that the processor included in the flavor inhaler or the like 1400 executes by using the sensor 1420. This step is preferably executed when the heater 121A or the heater 121B is not active for safety.

Reference sign 1720 indicates a step of converting input data indicating the detected motion of the flavor inhaler or the like 1400 to function data for controlling the function entity 1410 included in the flavor inhaler or the like 1400. This step may be the one that the converter 1430 included in the flavor inhaler or the like 1400 executes. This step may be the one that the processor included in the flavor inhaler or the like 1400 executes as the converter 1430.

Reference sign 1730 indicates a step of controlling the function entity 1410 in accordance with the function data obtained in step 1420. This step may be the one that the controller 1440 included in the flavor inhaler or the like 1400 executes and may be the one including the above-described example process 1500 or example process 1600. This step may be the one that the processor included in the flavor inhaler or the like 1400 executes as the controller 1440. A step of controlling the function entity 1410 in accordance with function data may be executed during inhalation of the flavor inhaler or the like 1400 by a user, or may be executed in response to a predetermined operation (such as depressing a button) on the flavor inhaler or the like 1400 by a user, or may be executed at other various timings. After the controller 1440 creates function data, the controller 1440 may automatically control the function entity 1410 in accordance with the function data so that a user can check the function data.

Of course, a program may cause the processor of the flavor inhaler or the like 1400 to execute the control method 1700.

The power supply 111A or power supply 111B of the flavor inhaler or the like 1400 may include a rechargeable battery. In this case, the battery may be charged by a charging apparatus electrically connected to the flavor inhaler or the like 1400. The charging apparatus may include not only the charger but also at least one of a function entity, a sensor, a converter, a controller, and the like similar to the structural elements illustrated in Fig. 14. When the flavor inhaler or the like 1400 is connected to the charging apparatus as described above, at least some of the steps of the control method 1400 may be executed by the structural elements in the charging apparatus.

According to the second embodiment of the present invention, when a user moves the flavor inhaler or the like, a variety of function data is generated. It is possible to activate the structural elements of the flavor inhaler or the like in a variety of modes in accordance with the generated function data. For this reason, a user is able to inhale the flavor inhaler or the like while the user feels stimulation in a variety of modes including a mode that the user cannot predict. Therefore, it is possible to improve user experience.

3 Third Embodiment of Present Invention

3-1 Simplified Configuration Example

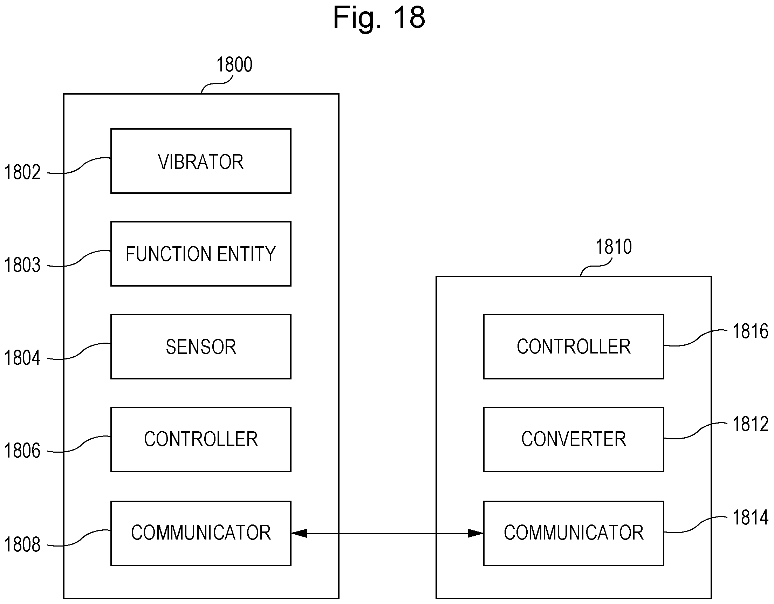

Fig. 18 is a schematic diagram that schematically illustrates a simplified configuration example of only structural elements particularly related to a third embodiment of the present invention and extracted.

Fig. 18 illustrates a flavor inhaler or the like 1800 and an external apparatus 1810 according to the present embodiment. The flavor inhaler or the like 1800 includes a vibrator 1802, a function entity 1803, a sensor 1804, a controller 1806, and a communicator 1808. The flavor inhaler or the like 1800 may include both the vibrator 1802 and the function entity 1803 or may include only any one of the vibrator 1802 and the function entity 1803. The flavor inhaler or the like 1800 may be the flavor inhaler or the like 100A or the flavor inhaler or the like 100B or may be another flavor inhaler or the like including the components illustrated in Fig. 18.

The vibrator 1802 may have components, functions, and the like similar to those of the vibrator 210 described in relation to Fig. 2. The function entity 1803 may have components, functions, and the like similar to those of the function entity 1410 described in relation to the second embodiment. The sensor 1804 may have components, functions, and the like similar to those of the sensor 220 described in relation to Fig. 2 or the sensor 1420 described in relation to the second embodiment. The sensor 1804 detects the motion of the flavor inhaler or the like 1800. Input data indicating the motion may be stored in a memory (not illustrated) of the flavor inhaler or the like 1800.

The communicator 1808 may include a communication interface (including a communication module) that conforms with a predetermined LPWA wireless communication standard or a wireless communication standard having similar limitations. Sigfox, LoRA-WAN, or the like can be adopted as such a communication standard. The communicator 1808 is a communication interface capable of communication that conforms with any wired or wireless communication standard. For example, Wi-Fi (registered trademark), Bluetooth (registered trademark), or the like can be adopted as such a communication standard. The flavor inhaler or the like 1800 is capable of communicating with the external apparatus 1810 via the communicator 1808. For example, the communicator 1808 sends input data indicating the motion of the flavor inhaler or the like 1800, detected by the sensor 1804, to the external apparatus 1810. The communicator 1808 also receives, from the external apparatus 1810, vibration data for vibrating the vibrator 1802 or function data for controlling the function entity 1803, obtained by converting input data in the external apparatus 1810.

The controller 1806 may be regarded as the one excluding the converter 230 or the converter 1430 from the controller 116A or the controller 116B. The controller 1806 is configured to vibrate the vibrator 1802 in accordance with vibration data received from the external apparatus 1810 or control the function entity 1803.

The external apparatus 1810 includes a converter 1812 and a communicator 1814. The external apparatus 1810 includes a controller 1816 configured to control structural elements in the external apparatus 1810, that is, the converter 1812, the communicator 1814, and the like.

The external apparatus 1810 may be any one of a personal computer (PC), a server, a mobile phone (including a smartphone), a tablet computer, a personal digital assistant, a wearable computer, and other various devices, configured to be capable of communicating with the flavor inhaler or the like 1800. The external apparatus 1810 may be an apparatus for charging the flavor inhaler or the like 1800 and may have a charger configured to charge a rechargeable battery in the flavor inhaler or the like 1800.

The communicator 1814 may have a function similar to the communicator 1808. The external apparatus 1810 is capable of communicating with the flavor inhaler or the like 1800 via the communicator 1814. For example, the communicator 1814 receives input data indicating the motion of the flavor inhaler or the like 1800, detected by the sensor 1804, from the flavor inhaler or the like 1800.

The converter 1812 may have a function similar to the converter 230 or the converter 1430. The converter 1812 is configured to convert input data received by the communicator 1814 to vibration data for vibrating the vibrator 1802 or function data for controlling the function entity 1803. Input data and vibration data or function data may be stored in a memory (not illustrated) in the external apparatus 1810. Input data and vibration data or function data may be editable by the external apparatus 1810. The communicator 1814 sends vibration data or function data to the flavor inhaler or the like 1800. The communicator 1814 may also send vibration data or function data to various devices capable of communicating with the external apparatus 1810 and different from the flavor inhaler or the like 1800. For example, when a user of the external apparatus 1810 sends favorite vibration data or function data stored in the memory of the external apparatus 1810 to a friend's smartphone or the like, the user is able to share the vibration data or the function data with the friend.

The sensor 1804 and input data are respectively similar to the sensor 220 described in Section 1-4 or the sensor 1420 described in relation to the second embodiment and input data, so the detailed description is omitted here.

The converter 1812 is similar to the converter 230 described in Section 1-5 or the converter 1430 described in relation to the second embodiment except that the converter 1812 is disposed in the external apparatus 1810. Vibration data that is generated by the converter 1812 is similar to vibration data described in Section 1-5. Function data that is generated by the converter 1812 is similar to function data described in relation to the second embodiment. Therefore, here, the detailed description of the converter 1812, vibration data, and function data is omitted.

The controller 1806 is similar to the controller 240 described in Section 1-6 or the controller 1440 described in relation to the second embodiment, so the detailed description is omitted here.

3-2 Control Method for Flavor Inhaler or the Like 1800 and External Apparatus 1810

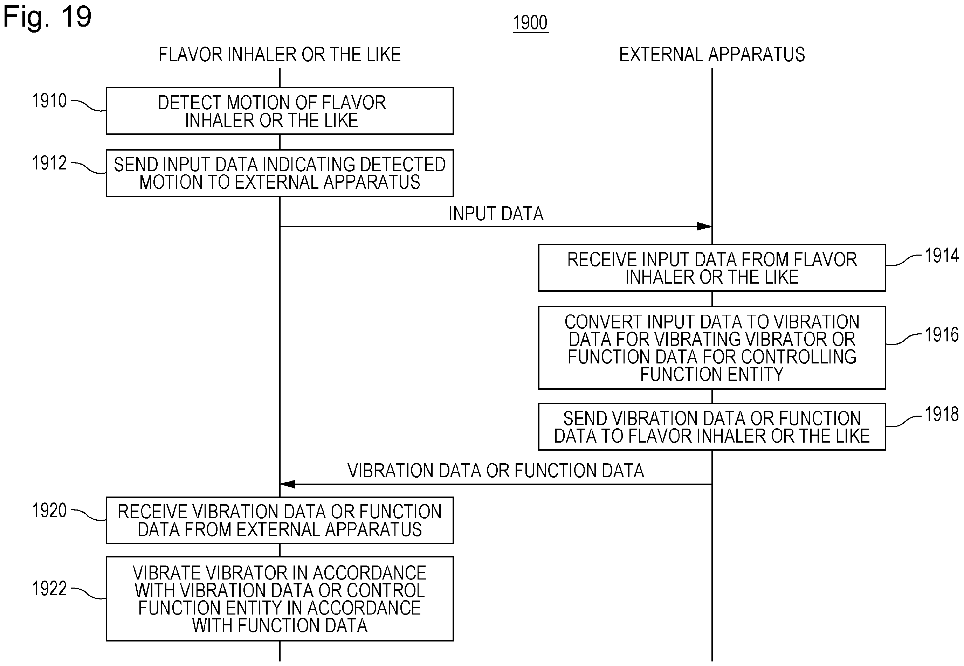

Fig. 19 is a sequence diagram that illustrates the operation of the flavor inhaler or the like 1800 and the operation of the external apparatus 1810 according to the present embodiment.

In step 1910, the flavor inhaler or the like 1800 detects the motion of the flavor inhaler or the like 1800. Step 1910 may be, for example, executed by the sensor 1804 or may be executed by the controller 1806 via the sensor 1804.

In step 1912, the flavor inhaler or the like 1800 sends input data indicating the detected motion to the external apparatus 1810. Step 1912 may be, for example, executed by the communicator 1808 or may be executed by the controller 1806 via the communicator 1808.

In step 1914, the external apparatus 1810 receives input data from the flavor inhaler or the like 1800. Step 1914 may be, for example, executed by the communicator 1814 or may be executed by the controller 1816 via the communicator 1814.

In step 1916, the external apparatus 1810 converts the input data to vibration data for vibrating the vibrator 1802 in the flavor inhaler or the like 1800 or function data for controlling the function entity 1803 in the flavor inhaler or the like 1800. Step 1916 may be, for example, executed by the converter 1812 or may be executed by the controller 1816 via the converter 1812.

In step 1918, the external apparatus 1810 sends the vibration data or the function data to the flavor inhaler or the like 1800. Step 1918 may be, for example, executed by the communicator 1814 or may be executed by the controller 1816 via the communicator 1814.

In step 1920, the flavor inhaler or the like 1800 receives the vibration data or the function data from the external apparatus 1810. Step 1920 may be, for example, executed by the communicator 1808 or may be executed by the controller 1806 via the communicator 1808.

In step 1922, the flavor inhaler or the like 1800 vibrates the vibrator 1802 in accordance with the vibration data or controls the function entity 1803 in accordance with the function data. Step 1922 may be executed by the controller 1806.

A program stored in the memory or the like of the flavor inhaler or the like 1800 may cause the flavor inhaler or the like 1800 to execute step 1910, step 1912, step 1920, and step 1922.

A program stored in the memory or the like of the external apparatus 1810 may cause the external apparatus 1810 to execute step 1914, step 1916, and step 1918.

According to the third embodiment of the present invention, at least part of work (for example, conversion from input data to vibration data or function data) of work that is executed in the flavor inhaler or the like is executed in the external apparatus in the first embodiment or the second embodiment. Therefore, it is possible to simplify the configuration of the flavor inhaler or the like. It is also possible to store created various pieces of vibration data or function data in the external apparatus and manage the various pieces of vibration data or function data.

4 Fourth Embodiment of Present Invention

Different from the above-described embodiments or in addition to the above-described embodiments, the flavor inhaler or the like according to the present invention may be configured to, when the sensor is acquiring input data indicating a detected motion of the flavor inhaler or the like, activate at least one sensory stimulation element that imparts a sensory stimulation to the user.

In the following description related to the fourth embodiment, "sensory stimulation data" includes vibration data in the first embodiment or function data in the second embodiment, "sensory stimulation strength" includes a vibration strength in the first embodiment or a function strength in the second embodiment, "sensory stimulation pattern" includes a vibration pattern in the first embodiment or a function pattern in the second embodiment, "sensory stimulation time" includes a vibration time in the first embodiment or a function time in the second embodiment, "sensory stimulation stop time" includes a vibration stop time in the first embodiment or a function stop time in the second embodiment, and "sensory stimulation strength correction coefficient" includes a vibration strength correction coefficient in the first embodiment or a function strength correction coefficient in the second embodiment. The details and deriving methods of sensory stimulation data, sensory stimulation strength, sensory stimulation pattern, sensory stimulation time, sensory stimulation stop time, and sensory stimulation strength correction coefficient in the fourth embodiment may be similar to the details and deriving methods of vibration data, vibration strength, vibration pattern, vibration time, vibration stop time, and vibration strength correction coefficient in the first embodiment or the details and deriving methods of function data, function strength, function pattern, function time, function stop time, and function strength correction coefficient in the second embodiment.

4-1 Simplified Configuration Example

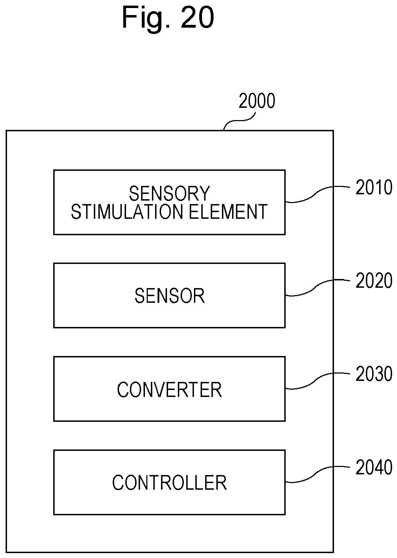

Fig. 20 is a schematic diagram that schematically illustrates a simplified configuration example of only structural elements particularly related to the present embodiment and extracted from the above-described flavor inhaler or the like 100A or flavor inhaler or the like 100B. Therefore, reference sign 2000 indicates the flavor inhaler or the like 100A or the flavor inhaler or the like 100B.

The flavor inhaler or the like 2000 includes at least one element 2010 (hereinafter, also referred to as "sensory stimulation element 2010") configured to impart a sensory stimulation to a user, a sensor 2020, a converter 2030, and a controller 2040.

The sensory stimulation element 2010 is the above-described vibrator 210 or function entity 1410, which is capable of imparting a sensory stimulation. Therefore, the sensory stimulation element 2010 is, for example, the vibrator 210, the light-emitting device or light-emitting element included in the notifier 113A or the notifier 113B, the display device that shows an image and that is included in the notifier 113A or the notifier 113B, or the sound output device or acoustic element that outputs sound and that is included in the notifier 113A or the notifier 113B; however, the sensory stimulation element 2010 is not limited thereto.

The sensor 2020 is the above-described sensor 220 or sensor 1420. The sensor 2020 is configured to detect the motion of the flavor inhaler or the like 2000. The sensor 2020 operates as described in the above embodiments. Furthermore, the sensor 2020 may be configured to acquire input data when generation of a flavor or aerosol by heating is not being performed in the flavor inhaler or the like 2000. As in the case of the above-described embodiments, input data may include data indicating an acceleration or angular velocity of the detected motion.

The controller 2040 is the above-described controller 240 or controller 1440. The controller 2040 is configured to activate the sensory stimulation element 2010 when the sensor 2020 is acquiring input data indicating the detected motion. The controller 2040 operates as described in the above embodiments. The sensory stimulation element 2010 may include two or more sensory stimulation elements configured to impart a sensory stimulation to a user. In this case, the controller 2040 may be configured to, while the sensor 2020 is in operation, further activate the sensory stimulation element different from the above-described at least one sensory stimulation element of the two or more sensory stimulation elements.

The converter 2030 is the above-described converter 230 or converter 1430. The converter 2030 is configured to convert input data to sensory stimulation data for activating the sensory stimulation element 2010.

As described in relation to the first embodiment and the second embodiment, the converter 2030 may be configured to convert the value of input data directly to a sensory stimulation strength. In this case, it is possible to directly impart the motion of the flavor inhaler or the like 2000, detected by the sensor 2020, to a user in real time. Input data may be data indicating the one obtained by combining motions on multiple axes of the flavor inhaler or the like 200. Such a combination may be, for example, the sum of values detected by the sensor 2020 at the same time or timing on each of the multiple axes.

As described in relation to the first embodiment and the second embodiment, the converter 2030 may be configured to use a representative value included in input data. As described in relation to the first embodiment and the second embodiment, using a representative value included in input data may include converting a representative value to a sensory stimulation strength (strength related to a sensory stimulation) of the sensory stimulation element 2010. As described in relation to the first embodiment and the second embodiment, using a representative value included in input data may include converting a representative value to a sensory stimulation pattern (pattern related to sensory stimulation) of the sensory stimulation element 2010.

As described in relation to the first embodiment and the second embodiment, the converter 2030 may be configured to divide input data to a plurality of pieces of data each representing a motion detected in each of a plurality of time periods. In addition, the converter 2030 may be configured to convert a representative value included in each divided piece of data to a sensory stimulation strength of the sensory stimulation element 2010 at different timing.

As described in relation to the first embodiment and the second embodiment, the sensor 2020 may be configured to detect at least the motion of the flavor inhaler or the like 2000 along at least a first axis and the motion of the flavor inhaler or the like 2000 along a second axis. Input data may include at least first input data representing the detected motion along the first axis and second input data representing the detected motion along the second axis.

As described in relation to the first embodiment and the second embodiment, the converter 2030 may be configured to convert first input data to a sensory stimulation strength of the sensory stimulation element 2010 and convert second input data to a sensory stimulation pattern of the sensory stimulation element 2010 or select one of a plurality of predetermined sensory stimulation patterns as a sensory stimulation pattern of the sensory stimulation element 2010 in accordance with second input data.

As described in relation to the first embodiment and the second embodiment, a sensory stimulation pattern may include at least one of a sensory stimulation time (a time during which the sensory stimulation element is active), a sensory stimulation stop time (a time during which the sensory stimulation element is inactive), and a sensory stimulation strength correction coefficient (a correction coefficient of a strength associated with sensory stimulation).

4-2 Control Method for Flavor Inhaler or the Like 2000

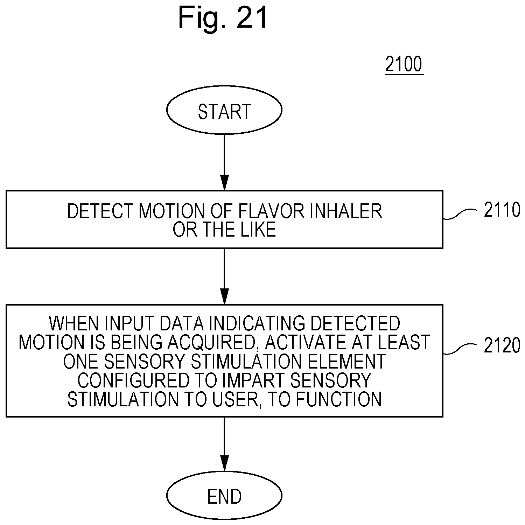

Fig. 21 is a flowchart of a control method 2100 for the flavor inhaler or the like 2000.

The control method 2100 may be started in response to startup of an input mode of the flavor inhaler or the like 2000. The input mode of the flavor inhaler or the like 2000 may be started up in response to, for example, a situation in which a user depresses a button provided for the flavor inhaler or the like 2000, a situation in which a user shakes the flavor inhaler or the like 2000 a predetermined number of times (for example, three times within a predetermined time), a situation in which instructions for mode change are received from an external apparatus, such as a smartphone, that communicates with the flavor inhaler or the like 2000, or the like. A user may be notified of startup and termination of the input mode by activating the vibrator, the acoustic element, the light-emitting element, or the like, provided in the flavor inhaler or the like 2000.

The flavor inhaler or the like 2000 may be configured not to execute the control method 2100 while electric power is being supplied to the heater 121A or the heater 121B. The flavor inhaler or the like 2000 may be configured not to execute the control method 2100 while the stick substrate 150 is inserted in the flavor inhaler or the like 100B.

In step 2110, the sensor 2020 detects the motion of the flavor inhaler or the like 2000. Step 2110 may be executed by the sensor 2020 or may be executed by the controller 2040 via the sensor 2020.

In step 2120, the controller 2040 activates the sensory stimulation element 2010 when the controller 2040 is acquiring input data indicating the detected motion. In an example, when the sensory stimulation element 2010 includes a light-emitting element, the controller 2040 may light the light-emitting element in a mode (such as a color and a blinking interval) different from a lighting mode of the light-emitting element while electric power is being supplied to the heater 121A or the heater 121B in step 2120. During the input mode, how the sensory stimulation element 2010 is activated at the time when the motion of the flavor inhaler or the like 2000 is detected may differ from how the sensory stimulation element 2010 is activated at the time when the motion of the flavor inhaler or the like 2000 is not detected. When, for example, the sensory stimulation element 2010 includes a vibrator or an acoustic element in addition to the light-emitting element, the light-emitting element may be constantly lit during the input mode; whereas the vibrator or the acoustic element may be additionally activated only when the motion of the flavor inhaler or the like 2000 is detected during the input mode.

In process 2100, the length of a period during which input data is acquired may be predetermined. The length of a period during which input data is acquired may be selected from among a plurality of preset candidates. The length of a period during which input data is acquired may be set by a user operating the button of the flavor inhaler or the like 2000, sending instructions from an external apparatus, or the like. A period during which input data is acquired may be one to 20 seconds and preferably may be three to 10 seconds.

A program stored in the memory or the like of the flavor inhaler or the like 2000 may cause the flavor inhaler or the like 2000 to execute the control method 2100.

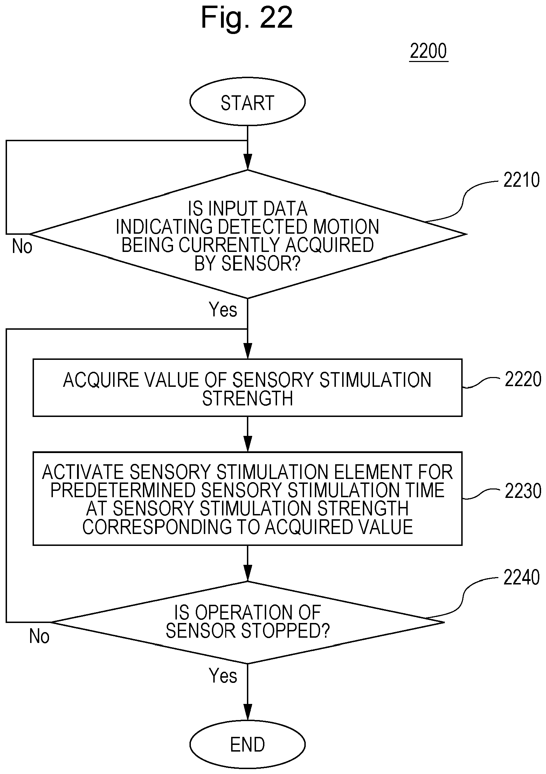

Fig. 22 is a flowchart that illustrates an example of operations that are executed by the controller 2040 of the flavor inhaler or the like 2000.

In step 2210, the controller 2040 determines whether the input data indicating the detected motion is currently being acquired by the sensor 2020. When the input data indicating the detected motion is currently not being acquired by the sensor 2020 (No in step 2210), the process returns to step 2210. When the input data indicating the detected motion is currently being acquired by the sensor 2020 (Yes in step 2210), the process proceeds to step 2220.

In step 2220, the controller 2040 acquires the value of sensory stimulation strength, included in sensory stimulation data of the sensory stimulation element 2010. Since step 2220 is similar to step 720 of Fig. 7 or step 1520 of Fig. 15, the detailed description is omitted here.

In step 2230, the controller 2040 activates the sensory stimulation element 2010 for a predetermined sensory stimulation time at the sensory stimulation strength corresponding to the acquired value. Since step 2230 is similar to step 730 or step 1530, the detailed description is omitted here.

In step 2240, the controller 2040 determines whether the operation of the sensor 2020 is stopped. When the operation of the sensor 2020 is stopped (Yes in step 2240), the process ends. When the operation of the sensor 2020 is not stopped (No in step 2240), the process returns to step 2220.

A program stored in the memory or the like of the flavor inhaler or the like 2000 may cause the flavor inhaler or the like 2000 to execute the process 2200.

With the process 2200, when inhalation of the flavor inhaler or the like is not performed, it is possible to impart a sensory stimulation to a user by activating the sensory stimulation element while the user is performing an action, such as shaking the flavor inhaler or the like. Thus, it is possible to cause the user to perceive that the flavor inhaler or the like is acquiring input data in accordance with user's action.

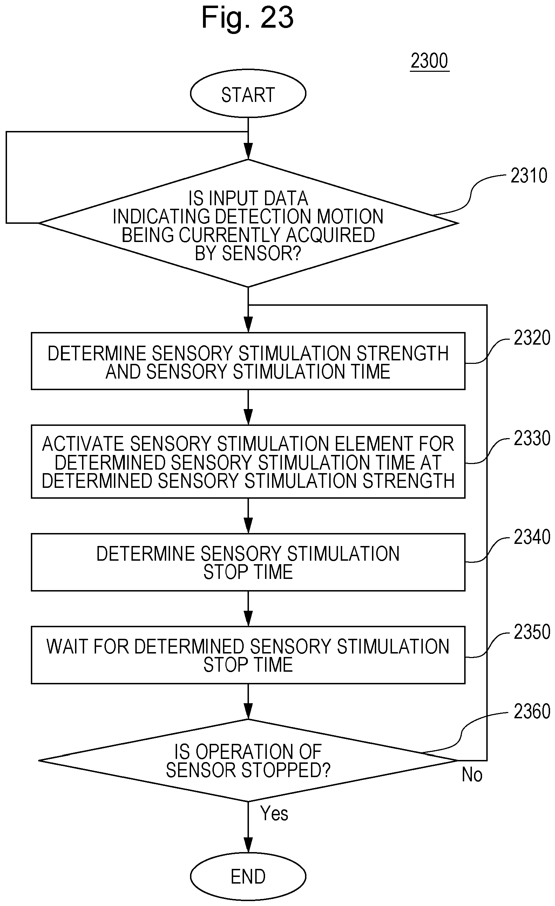

Fig. 23 is a flowchart that illustrates an example of operations that are executed by the controller 2040 of the flavor inhaler or the like 2000.

The process of step 2310 is similar to the process of step 2210.

In step 2320, the controller 2040 determines a sensory stimulation strength and a sensory stimulation time at the time of activating the sensory stimulation element 2010. Since step 2320 is similar to step 820 of Fig. 8 or step 1620 of Fig. 16, the detailed description is omitted here.

In step 2330, the controller 2040 activates the sensory stimulation element 2010 for the determined sensory stimulation time at the determined sensory stimulation strength. Since step 2330 is similar to step 830 or step 1630, the detailed description is omitted here.

In step 2340, the controller 2040 determines the sensory stimulation stop time of the sensory stimulation element 2010. Since step 2340 is similar to step 840 or step 1640, the detailed description is omitted here.

In step 2350, the controller 2040 causes the sensory stimulation element 2010 to wait for the determined sensory stimulation stop time. Since step 2350 is similar to step 850 or step 1650, the detailed description is omitted here.

In step 2360, the controller 2040 determines whether the operation of the sensor 2020 is stopped. When the operation of the sensor 2020 is stopped (Yes in step 2360), the process ends. When the operation of the sensor 2020 is not stopped (No in step 2360), the process returns to step 2320.

A program stored in the memory or the like of the flavor inhaler or the like 2000 may cause the flavor inhaler or the like 2000 to execute the process 2300.

With the process 2300, when inhalation of the flavor inhaler or the like is not performed, it is possible to impart a sensory stimulation to a user by activating the sensory stimulation element while the user is performing an action, such as shaking the flavor inhaler or the like. Thus, it is possible to cause the user to perceive that the flavor inhaler or the like is acquiring input data in accordance with user's action.

According to the fourth embodiment of the present invention, when a user moves a flavor inhaler or the like, a variety of sensory stimulation data is generated. While the user is moving the flavor inhaler or the like, the sensory stimulation element of the flavor inhaler or the like can be activated in a variety of modes in accordance with the generated sensory stimulation data. Therefore, the user is able to perceive that, for example, the motion of the flavor inhaler or the like is being detected by the sensor, input data indicating the motion is being generated, or sensory stimulation data is being generated in accordance with the input data. In addition, the user is able to feel stimulation in a variety of modes including a mode that the user cannot predict even when the user is not inhaling the flavor inhaler or the like. Therefore, it is possible to improve user experience.

5 Fifth Embodiment of Present Invention

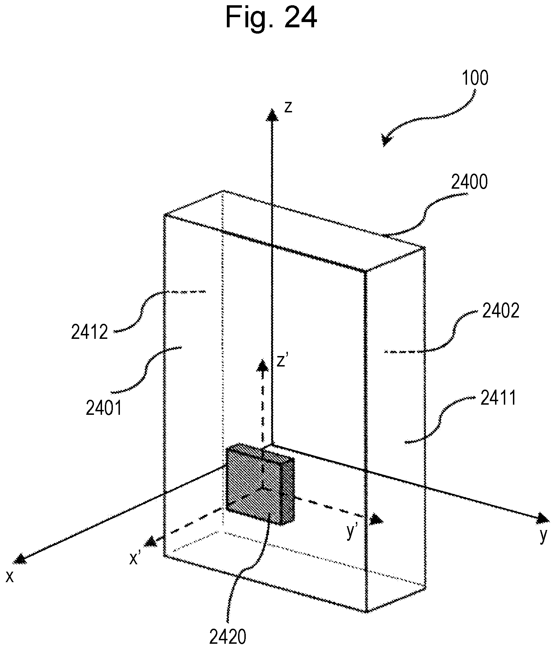

Hereinafter, a hardware configuration example of the flavor inhaler or the like according to each of the above-described embodiments will be described. Fig. 24 is a diagram that illustrates an example of the hardware configuration of a flavor inhaler or the like 100 (100A, 100B) and particularly a diagram that illustrates an example of a positional relationship between a casing 2400 and a sensor 2420 of the flavor inhaler or the like 100.

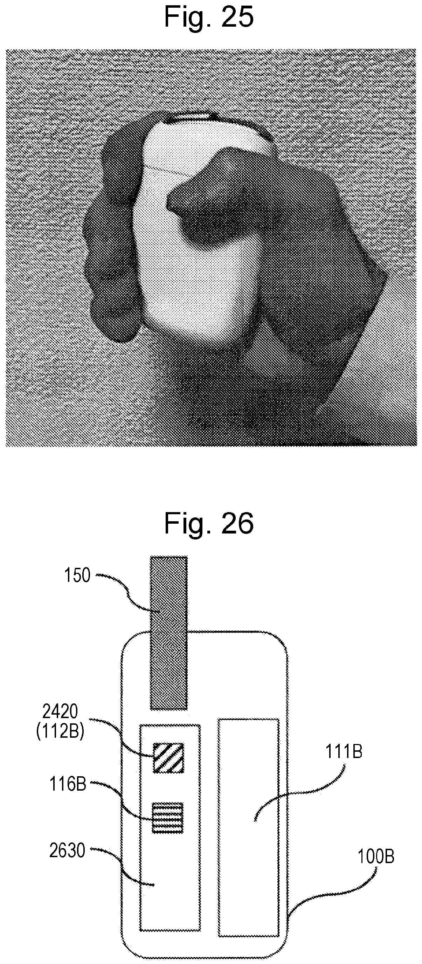

In this example, the casing 2400 of the flavor inhaler or the like 100 has a thick substantially rectangular parallelepiped shape having a pair of two substantially rectangular faces 2401, 2402 substantially parallel to each other. In Fig. 24, an X-axis, a Y-axis, and a Z-axis that are three coordinate axes of a three-dimensional coordinate system (right-handed coordinate system) in the casing 2400 are represented by continuous lines, and an X'-axis, a Y'-axis, and a Z'-axis that are three coordinate axes of a three-dimensional coordinate system (right-handed coordinate system) in the sensor 2420 are indicated by dashed lines. For example, on the assumption that a user holds the flavor inhaler or the like 100 illustrated in Fig. 24 so as to sandwich (in a Y-axis direction) the flavor inhaler or the like 100 by touching substantially parallel two faces 2411, 2412 that are thickness parts of the substantially rectangular parallelepiped shape of the casing 2400 with a thumb and a finger other than the thumb, such as an index finger, as illustrated in Fig. 25, a motion around the X-axis of the casing 2400 can correspond to an axis for detecting an up and down motion of a wrist of the user at the time when the user holds the flavor inhaler or the like 100, a motion around the Z-axis can correspond to an axis for detecting an in and out motion of the wrist of the user, and a motion around the Y-axis can correspond to an axis for detecting a motion of twisting of the wrist of the user. Here, the phrase "twisting of the wrist" means a motion to rotate a hand (wrist) around an axis in a direction heading from an elbow of the user to the wrist. The phrase "up and down of the wrist" means a motion to rotate the wrist around an axis in a direction perpendicular to the palm of the hand (or a motion to, in a state where the hand is oriented such that the little finger is placed at the bottom and the index finger is placed at the top, move the entire arm about the elbow as a rotation axis). The phrase "in and out (motion) of the wrist" means a motion to fold the wrist inward or extend the wrist outward around an axis in a direction substantially perpendicular to the direction from the elbow of the user toward the wrist and parallel to the palm of the hand (hereinafter, the same applies). In this example, the three-dimensional coordinate system in the casing 2400 and the three-dimensional coordinate system in the sensor 2420 will be described as right-handed coordinate systems; however, the configuration is not limited thereto. The three-dimensional coordinate systems may be left-handed coordinate systems or may be different from each other.

In this example, three coordinate axes in the casing 2400 are respectively set such that the longitudinal direction of the substantially rectangular face of the casing 2400 is a Z-axis, the widthwise direction of the substantially rectangular face is a Y-axis, and a direction orthogonal to the Z-axis and the Y-axis (a direction perpendicular to the substantially rectangular face and corresponding to the thickness direction of the substantially rectangular parallelepiped shape) is an X-axis. Then, in Fig. 24, the casing 2400 and the sensor 2420 are disposed such that the three coordinate axes, that is, the X-axis, the Y-axis, and the Z-axis, in the casing 2400 are respectively substantially parallel to three coordinate axes, that is, the X'-axis, the Y'-axis, and the Z'-axis, in the sensor 2420. In this way, when the orientations of the three coordinate axes in the casing 2400 coincide with the orientations of the three coordinate axes in the sensor 2420, vibrations (data) detected by the sensor 2420 are directly vibrations (data) of the casing 2400, and the process for detecting vibrations is simplified, so it is preferable. However, the configuration is not limited thereto. When the orientations of three coordinate axes in the casing 2400 do not coincide with the orientations of three coordinate axes in the sensor 2420, but the positional relationship between the three coordinate axes of the casing 2400 and the three coordinate axes of the sensor 2420 is known in advance. Therefore, it is possible to calculate the orientation and strength of vibrations of the casing 2400 by incorporating data indicating a difference produced from the positional relationship into the vibration data detected by the sensor 2420.

The example of Fig. 24 is only illustrative, and, for example, any one of the three coordinate axes, that is, the X'-axis, the Y'-axis, and the Z'-axis, in the sensor 2420 may be disposed substantially parallel to any one of the three coordinate axes, that is, the X-axis, the Y-axis, and the Z-axis, in the casing 2400. In this case as well, for the coordinate axes of the casing 2400 and the coordinate axes of the sensor 2420, disposed substantially parallel to each other, no complicated calculation is needed for vibration data detected by the sensor 2420, so processing on vibrations of the flavor inhaler or the like 100 can be reduced.

The sensor 2420 is included in the sensor 112A of Fig. 1A or the sensor 112B of Fig. 1B, and the sensor 2420 in Fig. 24 may be regarded as the sensor 112A or the sensor 112B. Examples of the sensor 2420 can include the above-described sensor 220, sensor 1420, and sensor 1804.

In the description of Fig. 24, the term "substantially" means to allow not strict coincidence (hereinafter, the same applies). For example, the phrase "substantially parallel" or the phrase "substantially perpendicular" means to allow a certain amount of deviation from a parallel position or a perpendicular position. The deviation may be, for example, about 10°.

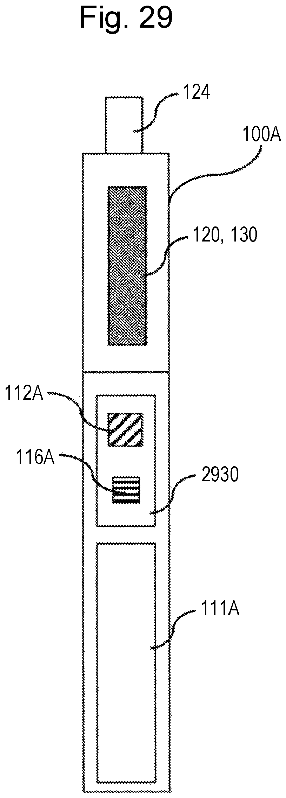

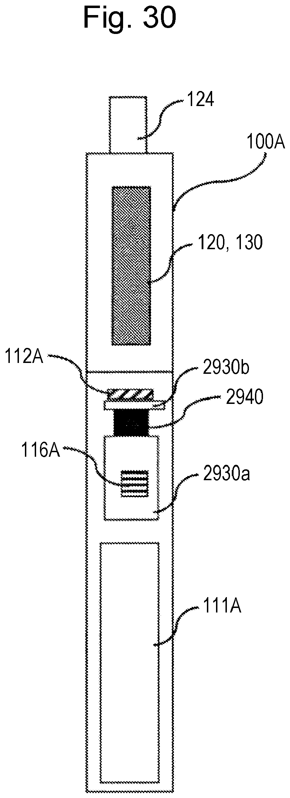

Next, an arrangement example of the sensor 2420 in the flavor inhaler or the like 100 will be described. Figs. 26 to 30 are diagrams that illustrate arrangement examples of the sensor 2420 in the flavor inhaler or the like 100. Figs. 26 to 28 illustrate the flavor inhaler or the like 100B of Fig. 1B as an example. Figs. 29 and 30 illustrate the flavor inhaler or the like 100A of Fig. 1A as an example.

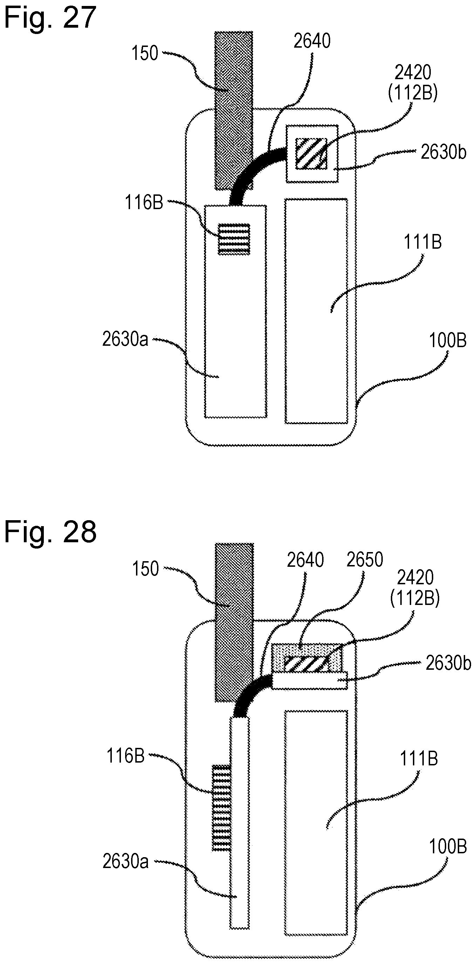

In Fig. 26, the stick substrate 150, the battery 111B (which corresponds to the power supply 111B of Fig. 1B; the same applies below), the sensor 2420 (sensor 112B), and the microcontroller 116B (which corresponds to the controller 116B of Fig. 1B; the same applies below) of the flavor inhaler or the like 100B are illustrated. The sensor 2420 (112B) is disposed at a location not in contact with the heater for heating the stick substrate 150 in the casing of the flavor inhaler or the like 100B (the same applies in Figs. 27 to 30 (described later)).

In Fig. 26, the sensor 2420 (112B) and the microcontroller 116B are disposed on the same printed circuit board 2630. On the other hand, in Figs. 27 and 28, the sensor 2420 (112B) and the microcontroller 116B are respectively disposed on different printed circuit boards 2630. Specifically, a first printed circuit board 2630a and a second printed circuit board 2630b are connected to each other by a flexible circuit board 2640, the microcontroller 116B is disposed on the first printed circuit board 2630a, and the sensor 2420 (112B) is disposed on the second printed circuit board 2630b.

Here, the sensor 2420 (112B) is allowed to be disposed at various locations of the flavor inhaler or the like 100B. However, when the sensor 2420 (112B) is disposed at a location closer to a user than the battery 111B (a location closer to the opening 142) at the time when the user inhales a material generated by the flavor inhaler or the like 100B as illustrated in Figs. 26 and 27, a specific motion (motion) of the flavor inhaler or the like 100B by the user is presumably easily detected.

Initially, the flavor inhaler or the like 100B is a device for a user to inhale a material, such as an aerosol, generated by the flavor inhaler or the like 100B, so it is estimated that the user usually holds the flavor inhaler or the like 100B such that the user easily puts the stick substrate 150, inserted in the opening 142 so as to be easily inhaled, in his or her mouth. In other words, it is estimated that the user holds the flavor inhaler or the like 100B such that the opening 142 is oriented upward during inhalation. At this time, the position of the wrist (or the elbow) of the user is lower than the position of the opening 142. Then, when, for example, the user shakes the flavor inhaler or the like 100B, the sensor 2420 (112B) is placed farther from the wrist (or the elbow) of the user as the sensor 2420 (112B) approaches the opening 142, so vibrations imparted to the flavor inhaler or the like 100B are easily detected due to centrifugal force.

Furthermore, generally, it is estimated that the battery 111B is the heaviest among components and the like of the flavor inhaler or the like 100B, and the user is highly likely to hold a part near the battery 111B (or the center of gravity of the battery 111B) for the user to stably hold the flavor inhaler or the like 100B. Thus, since the sensor 2420 (112B) is disposed at a location closer to the opening 142 than the battery 111B (or the center of gravity of the battery 111B), when the user holds a part near the battery 111B and imparts vibrations to the flavor inhaler or the like 100B with an action, such as shaking the flavor inhaler or the like 100B, it is expected that the vibrations are more easily detected. In other words, it may be regarded that, since the sensor 2420 (112B) is disposed at a location closer to the opening 142 than the center of gravity of the flavor inhaler or the like 100B, vibrations that the user imparts to the flavor inhaler or the like 100B are more easily detected. The location of the sensor 2420 (112B) in the flavor inhaler or the like 100B, described above, is similar in Fig. 28 (described later)

In the examples of Figs. 26 and 27, as in the case of Fig. 24, the casing of the flavor inhaler or the like 100B may have a thick substantially rectangular parallelepiped shape having a pair of substantially rectangular two faces substantially parallel to each other, and the flavor inhaler or the like 100B and the sensor 2420 (112B) may be disposed such that the X'-axis, the Y'-axis, and the Z'-axis in the sensor 2420 (112B) are respectively substantially parallel to the X-axis, the Y-axis, and the Z-axis in the casing of the flavor inhaler or the like 100B. In this case, Figs. 26 and 27 and Fig. 28 (described later) are diagrams that illustrate the configuration inside the flavor inhaler or the like 100B when viewed in a direction facing one substantially rectangular face (which corresponds to one the face 2401 or the face 2402 of Fig. 24) of the flavor inhaler or the like 100B.

In the example illustrated in Fig. 28, as in the case of the example of Fig. 27, the sensor 2420 (112B) and the microcontroller 116B are respectively disposed on different printed circuit boards 2630. In other words, the microcontroller 116B is disposed on the first printed circuit board 2630a, and the sensor 2420 (112B) is disposed on the second printed circuit board 2630b. In the example of Fig. 28, the first printed circuit board 2630a and the microcontroller 116B are disposed so as to be perpendicular to the substantially rectangular face (the Y-Z plane of Fig. 24) of the casing of the flavor inhaler or the like 100B and parallel to the X-Z plane of Fig. 24. The second printed circuit board 2630b and the sensor 2420 (112B) are disposed so as to be perpendicular to the substantially rectangular face (the Y-Z plane of Fig. 24) of the casing of the flavor inhaler or the like 100B and parallel to the X-Y plane of Fig. 24. In this way, since the microcontroller 116B and/or the sensor 2420 (112B) is disposed so as to be perpendicular to the substantially rectangular face of the casing of the flavor inhaler or the like 100B, the area of each component on the substantially rectangular face of the flavor inhaler or the like 100B reduces, so it is possible to reduce the overall size of the flavor inhaler or the like 100B. As illustrated in Figs. 27 and 28, when the sensor 2420 (112B) and the microcontroller 116B are respectively disposed on the different printed circuit boards 2630a, 2630b, there is an advantage that, when the sensor 2420 (112B) is disposed at a location closer to a user than the battery 111B (a location closer to the opening 142 or the stick substrate 150) at the time when the user inhales a material generated by the flavor inhaler or the like 100B, it is possible to determine the location of the sensor 2420 (112B) without influence on the location or the size of the battery 111B.

In the sensor 2420 (112B) disposed on the second printed circuit board 2630b, at least part of a face on an opposite side to a face that is in contact with the second printed circuit board 2630b to which the sensor 2420 (112B) is attached may be covered with a heat insulator 2650. In the flavor inhaler or the like 100B, an aerosol is generated when the stick substrate 150 is heated by a heater (not illustrated); however, since at least part of the face on an opposite side to the face that is in contact with the second printed circuit board 2630b of the sensor 2420 (112B) is covered with the heat insulator 2650, it is possible to protect the sensor 2420 (112B) from heat generated by the heater. Thus, it is possible to reduce failure and malfunction of the sensor 2420 (112B).

Next, another arrangement example of the sensor 2420 in the flavor inhaler or the like 100 will be described with reference to Figs. 29 and 30. In Fig. 29, the cartridge 120 and the flavor imparting cartridge 130 (which are simply integrally illustrated in Fig. 29; the same applies in Fig. 30), the battery 111A (which corresponds to the power supply 111A of Fig. 1; hereinafter, the same applies), the sensor (sensor) 112A, and the microcontroller 116A (which corresponds to the controller 116A of Fig. 1; hereinafter, the same applies) of the flavor inhaler or the like 100A are illustrated . The sensor 112A and the microcontroller 116A are disposed on the same printed circuit board 2930. On the other hand, in Fig. 30, the sensor 112A and the microcontroller 116A are respectively disposed on different printed circuit boards 2930. Specifically, a first printed circuit board 2930a and a second printed circuit board 2930b are connected to each other by a flexible circuit board 2940, the microcontroller 116A is disposed on the first printed circuit board 2930a, and the sensor 112A is disposed on the second printed circuit board 2930b.

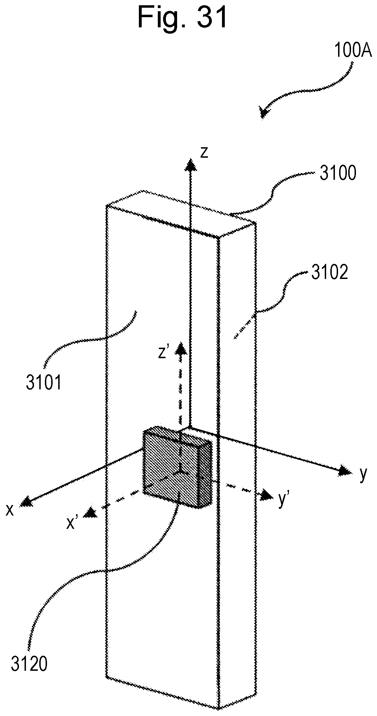

In the examples of Figs. 29 and 30 as well, as in the case of Fig. 24, the casing of the flavor inhaler or the like 100A may have a thick substantially rectangular parallelepiped shape having two substantially rectangular faces substantially parallel to each other. Fig. 31 is a diagram that illustrates the configuration of a case where a casing 3100 and a sensor 3120 are disposed in the flavor inhaler or the like 100A in a positional relationship similar to Fig. 29 as an example of such the substantially rectangular parallelepiped shape. Here, the sensor 3120 corresponds to the sensor 2420 (1112A) of Fig. 29. In the example illustrated in Fig. 31, a casing 3100 of the flavor inhaler or the like 100A has the thick substantially rectangular parallelepiped shape having a pair of two substantially rectangular faces 3101, 3102 substantially parallel to each other; however, the longitudinal lengths of the two substantially rectangular faces 3101, 3102 of the casing 3100 are longer several times than the widthwise lengths of the faces 3101, 3102, and the casing 3100 of the flavor inhaler or the like 100A has a long slender shape as a whole. In Fig. 31, an X-axis, a Y-axis, and a Z-axis that are three coordinate axes of a three-dimensional coordinate system (right-handed coordinate system) in the casing 3100 are represented by continuous lines, and an X'-axis, a Y'-axis, and a Z'-axis that are three coordinate axes of a three-dimensional coordinate system (right-handed coordinate system) in the sensor 3120 are indicated by dashed lines. For example, on the assumption that a user holds the flavor inhaler or the like 100A illustrated in Fig. 31 so as to sandwich (in an X-axis direction) the thickness parts of the casing 3100 by touching the two faces 3101, 3102 with a thumb and a finger other than the thumb, such as an index finger, a motion around the X-axis of the casing 3100 can correspond to an axis for detecting a motion of twisting of a wrist of the user at the time when the user holds the flavor inhaler or the like 100A, a motion around the Z-axis can correspond to an axis for detecting an in and out motion of the wrist of the user, and a motion around the Y-axis can correspond to an axis for detecting an up and down motion of the wrist of the user. In this example, the three-dimensional coordinate system in the casing 3100 and the three-dimensional coordinate system in the sensor 3120 will be described as right-handed coordinate systems; however, the configuration is not limited thereto. The three-dimensional coordinate systems may be left-handed coordinate systems or may be different from each other.

In the example of Fig. 30, the first printed circuit board 2930a and the microcontroller 116A are substantially parallel to the substantially rectangular face (the Y-Z plane of Fig. 24) of the casing of the flavor inhaler or the like 100A. The second printed circuit board 2930b and the sensor 112A are disposed so as to be perpendicular to the substantially rectangular surface (the Y-Z plane of Fig. 24) of the casing of the flavor inhaler or the like 100A and parallel to the X-Y plane of Fig. 24. However, in the example of Fig. 28, the first printed circuit board 2930a and the microcontroller 116A may be disposed so as to be perpendicular to the substantially rectangular face (the Y-Z plane of Fig. 24) of the casing of the flavor inhaler or the like 100A and parallel to the X-Z plane of Fig. 24. In this way, since the microcontroller 116A and/or the sensor 112A is disposed so as to be perpendicular to the substantially rectangular face of the casing of the flavor inhaler or the like 100A, the area of each component on the substantially rectangular face of the flavor inhaler or the like 100A reduces, so it is possible to reduce the overall size of the flavor inhaler or the like 100A. Since the microcontroller 116A and/or the sensor 112A is disposed so as to be perpendicular to the longitudinal direction of the flavor inhaler or the like 100A (parallel to the X-Y plane), the area of each component on the substantially rectangular face of the flavor inhaler or the like 100A reduces, so it is possible to reduce the overall size of the flavor inhaler or the like 100A.

In Figs. 29 and 30 as well, as described with reference to Figs. 26 to 28, when the sensor 112A is disposed at a location closer to a user than the battery 111A (a location closer to the mouthpiece 124) at the time when the user inhales a material generated by the flavor inhaler or the like 100A, it is presumable that a specific motion (motion) of the flavor inhaler or the like 100A by the user is presumably easily detected.

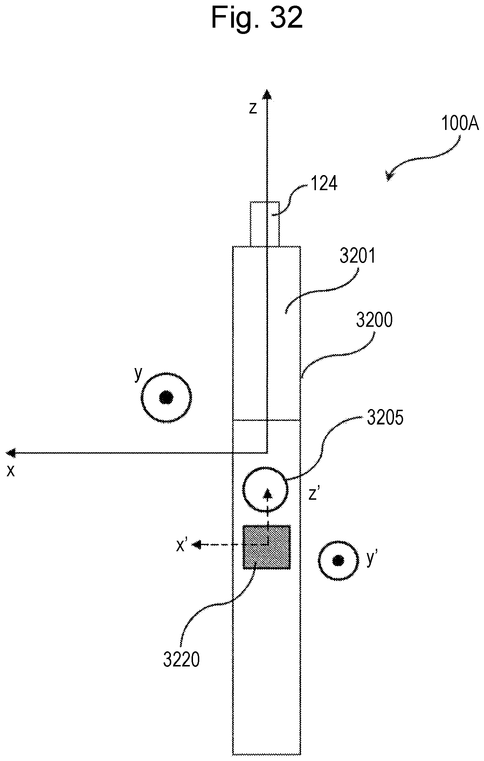

The flavor inhaler or the like 100A of Fig. 1A may have a substantially cylindrical shape as a whole. Fig. 32 is a diagram that illustrates an example of a front view in the case where the flavor inhaler or the like 100A has a long-slender substantially cylindrical shape. In the example of Fig. 32, the flavor inhaler or the like 100A has a substantially cylindrical casing 3200 and includes a button 3205 on the side for user's operation. Here, for example, it is assumed that the surface of the button 3205 is a substantially planar surface, a straight line direction orthogonal to this surface is a Y-axis of the casing 3200, and the longitudinal direction of the long-slender substantially cylindrical casing 3200 is a Z-axis, an X-axis direction is the widthwise direction of the casing 3200 (a diametrical direction of a bottom of the substantially cylindrical (substantially cylindrical columnar) shape) in the right-handed coordinate system. At this time, the sensor 3220 may be disposed such that an X-axis, a Y-axis, and a Z-axis that are three coordinate axes of a three-dimensional coordinate system (right-handed coordinate system) in the sensor 3220 are respectively substantially parallel to the X-axis, the Y-axis, and the Z-axis of the casing 3200, as indicated by the dashed lines in Fig. 32. In this way, when the orientations of the three coordinate axes in the casing 3200 coincide with the orientations of the three coordinate axes in the sensor 3220, vibrations (data) detected by the sensor 3220 are directly vibrations (data) of the casing 3200, and the process for detecting vibrations is further simplified, so it is preferable. However, the configuration is not limited thereto. The orientations of three coordinate axes in the casing 3200 do not need to coincide with the orientations of three coordinate axes in the sensor 3220.

On the assumption that a user holds the casing 3200 so as to sandwich the casing 3200 by touching the button 3205 with a thumb so that the user can operate the button 3205 with the thumb in Fig. 32, a motion around the X-axis of the casing 3200 can correspond to an axis for detecting an up and down motion of a wrist of the user at the time when the user holds the flavor inhaler or the like 100, a motion around the Z-axis can correspond to an axis for detecting an in and out motion of the wrist of the user, and a motion around the Y-axis can correspond to an axis for detecting a motion of twisting of the wrist of the user (however, this is one example, and which motions of the wrist of the user the axes respectively correspond to may be determined as needed, and the same applies to Figs. 24 and 31). When the flavor inhaler or the like 100A does not include the button 3205, and a logo or an LED is provided on the side 3201 of the substantially cylindrical casing 3200, three axes of the casing 3200 may be set as in the case of Fig. 32 on the assumption that the part is a planar surface. The term "substantially cylindrical shape" means that the casing 3200 just needs to have a substantially cylindrical shape as a whole and does not need to have a strict cylindrical shape.

In the above-described examples the sensor 2420 may be an inertial sensor (motion sensor), such as an acceleration sensor and an angular velocity sensor (gyro sensor). An axis that an acceleration sensor and an angular velocity sensor detect may be one to three axes. A detection range of the angular velocity sensor is not limited to a specific range; however, the detection range preferably ranges from $\pm 100$ dps to $\pm 5000$ dps and more preferably ranges from $\pm 300$ dps to $\pm 2000$ dps.

Up to here, the embodiments of the present invention have been described. The present invention is not limited to the above-described embodiments, and, of course, may be implemented in various different modes within the scope of the technical idea.

The scope of the present invention is not limited to example embodiments illustrated and described, and encompasses all the embodiments that lead to advantageous effects equivalent to those intended by the present invention. Furthermore, the scope of the present invention is not limited to combinations of features of the invention defined by the appended claims and can be defined by any desired combinations of specific features of disclosed all the features.

REFERENCE SIGNS LIST

| | |
|---|---|
| 100A, 100B, 200, 1400, 1800, 2000 | flavor inhaler or the like |
| 110 | power supply unit |
| 111A, 111B | power supply |
| 112A, 112B | sensor |
| 113A, 113B | notifier |
| 114A, 114B | memory |
| 115A, 115B, 1808, 1814 | communicator |
| 116A, 116B, 240, 1440, 1806, 1816, 2040 | controller |
| 117A, 117B, 230, 1430, 1812, 2030 | converter |
| 120 | cartridge |
| 121A,121B | heater |
| 122 | liquid guide |
| 123 | liquid storage |
| 124 | mouthpiece |
| 130 | flavor imparting cartridge |
| 131 | flavor source |
| 140 | holder |
| 141 | internal space |
| 142 | opening |
| 143 | bottom |
| 144 | heat insulator |
| 150 | stick substrate |
| 151 | substrate |
| 152 | inhalation port |
| 180 | airflow path |
| 181 | air inlet hole |
| 182 | air outlet hole |
| 210, 1802 | vibrator |
| 220, 1420, 1804, 2020, 2420, 3120, 3220 | sensor |
| 1410, 1803 | function entity |
| 1810 | external apparatus |
| 2010 | sensory stimulation element |
| 2400, 3100, 3200 | casing |
| 2630, 2630a, 2630b, 2930, 2930a, 2930b | printed circuit board |
| 2640, 2940 | flexible circuit board |
| 2650 | heat insulator |
| 3205 | button |

**Claims**

1. A device that is a flavor inhaler or an aerosol generating device, the device comprising:

    at least one sensory stimulation element configured to impart a sensory stimulation to a user;
    a sensor configured to detect a motion of the device; and
    a controller configured to, when the sensor is acquiring input data indicating the detected motion, activate the at least one sensory stimulation element.

2. The device according to claim 1, wherein the at least one sensory stimulation element includes at least one of a vibrator, a light-emitting element, and an acoustic element.

3. The device according to claim 1 or 2, wherein the input data includes data indicating an acceleration or angular velocity of the detected motion.

4. The device according to any one of claims 1 to 3, wherein the sensor is further configured to, when generation of an aerosol by heating is not being performed in the device, acquire the input data.

5. The device according to any one of claims 1 to 4, further comprising

    two or more sensory stimulation elements configured to impart a sensory stimulation to a user, wherein
    the controller is configured to, while the sensor is in operation, further activate the sensory stimulation element different from the at least one sensory stimulation element of the two or more sensory stimulation elements.

6. The device according to any one of claims 1 to 5, further comprising a converter configured to convert the input data to sensory stimulation data for activating the at least one sensory stimulation element.

7. The device according to claim 6, wherein the converter is further configured to use a representative value included in the input data.

8. The device according to claim 7, wherein using the representative value included in the input data includes converting the representative value to a strength associated with a sensory stimulation of the at least one sensory stimulation element.

9. The device according to claim 7, wherein using the representative value included in the input data includes converting the representative value to a pattern associated with a sensory stimulation of the at least one sensory stimulation element.

10. The device according to claim 8, wherein
    the converter is further configured to

    divide the input data to a plurality of pieces of data each representing the motion detected in a corresponding one of a plurality of time periods, and
    convert a representative value included in each of the divided pieces of data to a strength associated with a sensory stimulation of the at least one sensory stimulation element at different timing.

11. The device according to any one of claims 6 to 10, wherein

    the sensor is further configured to detect at least a motion of the device along a first axis and a motion of the device along a second axis, and
    the input data includes at least first input data representing the detected motion along the first axis and second input data representing the detected motion along the second axis.

12. The device according to claim 11, wherein
    the converter is further configured to

    convert the first input data to a strength associated with a sensory stimulation of the at least one sensory stimulation element, and

convert the second input data to a pattern associated with a sensory stimulation of the at least one sensory stimulation element or select one of a plurality of predetermined patterns associated with a plurality of sensory stimulations as a pattern associated with a sensory stimulation of the at least one sensory stimulation element in accordance with the second input data.

13. The device according to claim 9 or 12, wherein the pattern associated with the sensory stimulation is determined in accordance with at least one of a time during which the sensory stimulation element is active, a time during which the sensory stimulation element is inactive, and a correction coefficient of a strength associated with the sensory stimulation.

14. A control method for a device that is a flavor inhaler or an aerosol generating device, the control method comprising:

a step of detecting a motion of the device; and
a step of, when input data indicating the detected motion is being acquired, activating at least one sensory stimulation element configured to impart a sensory stimulation to a user.

15. A program causing a device that is a flavor inhaler or an aerosol generating device, to execute:
a step of detecting a motion of the device; and
a step of, when input data indicating the detected motion is being acquired, activating at least one sensory stimulation element configured to impart a sensory stimulation to a user.

Fig. 1A

100A

110

POWER SUPPLY UNIT

111A POWER SUPPLY
112A SENSOR
113A NOTIFIER
114A MEMORY
115A COMMUNICATOR

116A CONTROLLER

CONVERTER

117A

AIR

181

120 CARTRIDGE

122 121A 123

LIQUID STORAGE

130 FLAVOR IMPARTING CARTRIDGE

131

FLAVOR SOURCE

124 182

190

180

AIR

181

EP 4 413 884 A1

35

EP 4 413 884 A1

# Fig. 1B

# Fig. 2

200

| VIBRATOR | — 210 |
| SENSOR | — 220 |
| CONVERTER | — 230 |
| CONTROLLER | — 240 |

Fig. 3

Fig. 4

## Fig. 5

510A        510B        510C        510D

## Fig. 6A

| VIBRATION PATTERN | VIBRATION STRENGTH CORRECTION COEFFICIENT | | | | |
|---|---|---|---|---|---|
| 1 | 1 | - | - | - | - |
| 2 | 0.5 | 1 | - | - | - |
| 3 | 0.3 | 0.6 | 1 | - | - |
| 4 | 0.3 | 0.5 | 0.7 | 1 | - |
| 5 | 0.2 | 0.4 | 0.6 | 0.8 | 1 |

## Fig. 6B

| VIBRATION PATTERN | VIBRATION STRENGTH | | | | |
|---|---|---|---|---|---|
| 1 | 80 | - | - | - | - |
| 2 | 40 | 80 | - | - | - |
| 3 | 24 | 48 | 80 | - | - |
| 4 | 24 | 40 | 56 | 80 | - |
| 5 | 16 | 32 | 48 | 64 | 80 |

# Fig. 7

700

```
        ┌──────────┐
        │  START   │
        └────┬─────┘
             │
  ┌──────────┤
  │          ▼
  │    ◇─────────────────────◇ ─── 710
  │    │ IS INHALATION STARTED? │
  No ──◇─────────────────────◇
  │          │ Yes
  └──────────┤
             ▼
        ┌─────────────────────────────────┐ ─── 720
        │ ACQUIRE VALUE OF VIBRATION STRENGTH │
        └──────────────┬──────────────────┘
                       ▼
        ┌─────────────────────────────────┐ ─── 730
        │ VIBRATE VIBRATOR FOR PREDETERMINED │
        │ TIME AT VIBRATION STRENGTH        │
        │ CORRESPONDING TO ACQUIRED VALUE   │
        └──────────────┬──────────────────┘
                       ▼
   Yes          ◇─────────────────────◇ ─── 740
   ┌────────────│ IS INHALATION STOPPED? │
   │            ◇─────────────────────◇
   │                   │ No
   │                   ▼
   │            ◇─────────────────────◇ ─── 750
   │            │ VALUE OF VIBRATION    │
   │            │ STRENGTH CAN BE FURTHER│ ──── Yes
   │            │ ACQUIRED?             │
   │            ◇─────────────────────◇
   │                   │ No
   │                   ▼
   │            ┌──────────┐
   └───────────▶│   END    │
                └──────────┘
```

# Fig. 8A

800A

```
                    ┌─────────────┐
                    │    START    │
                    └──────┬──────┘
                           │
          ┌────────────────▼────────────────┐
          ◇      IS INHALATION STARTED?      ◇──── 810A
          └────────────────┬────────────────┘
                           │
        ┌──────────────────▼──────────────────┐
        │  DETERMINE VIBRATION STRENGTH AND    │──── 820A
        │         VIBRATION TIME               │
        └──────────────────┬──────────────────┘
                           │
        ┌──────────────────▼──────────────────┐
        │  VIBRATE VIBRATOR FOR DETERMINED     │──── 830A
        │  VIBRATION TIME AT DETERMINED        │
        │  VIBRATION STRENGTH                  │
        └──────────────────┬──────────────────┘
                           │
        ┌──────────────────▼──────────────────┐
        │   DETERMINE VIBRATION STOP TIME      │──── 840A
        └──────────────────┬──────────────────┘
                           │
        ┌──────────────────▼──────────────────┐
        │    WAIT FOR DETERMINED               │──── 850A
        │    VIBRATION STOP TIME               │
        └──────────────────┬──────────────────┘
                           │
          ┌────────────────▼────────────────┐
          ◇      IS INHALATION STOPPED?      ◇──── 860A
          └────────────────┬────────────────┘  No
                           │ Yes
                    ┌──────▼──────┐
                    │     END     │
                    └─────────────┘
```

# Fig. 8B

800B

START

IS INHALATION STARTED? —— 810B

No

Yes

ACQUIRE VALUES OF VIBRATION STRENGTH, VIBRATION TIME, AND VIBRATION STOP TIME —— 820B

VIBRATE VIBRATOR FOR ACQUIRED VIBRATION TIME AT ACQUIRED VIBRATION STRENGTH —— 830B

WAIT FOR ACQUIRED VIBRATION STOP TIME —— 840B

IS INHALATION STOPPED? —— 850B

Yes

No

VALUES OF VIBRATION STRENGTH, VIBRATION TIME, AND VIBRATION STOP TIME CAN BE FURTHER ACQUIRED? —— 860B

Yes

No

END

Fig. 9

900

910

EP 4 413 884 A1

# Fig. 10

1010A

1000A

1020A

1000B

EP 4 413 884 A1

EP 4 413 884 A1

# Fig. 11

1100

1110A

1100A

1120A

1100B

EP 4 413 884 A1

# Fig. 12A

1200A

1210A          1210A

1202A

1220A

1204A

# Fig. 12B

1210B

1202B

1220B

1204B

EP 4 413 884 A1

# Fig. 13

1300

START

DETECT MOTION OF FLAVOR INHALER OR THE LIKE — 1310

CONVERT INPUT DATA INDICATING DETECTED MOTION TO VIBRATION DATA — 1320

VIBRATE VIBRATOR IN ACCORDANCE WITH VIBRATION DATA — 1330

END

# Fig. 14

1400

FUNCTION ENTITY — 1410

SENSOR — 1420

CONVERTER — 1430

CONTROLLER — 1440

# Fig. 15

1500

```
           ┌─────────┐
           │  START  │
           └─────────┘
                │
                ▼
        ◇─────────────────◇ ─── 1510
  No    │ FUNCTION SHOULD  │
◄───────│  BE EXECUTED?    │
        ◇─────────────────◇
                │
               Yes
                │
                ▼
    ┌───────────────────────────────┐ ─── 1520
    │ ACQUIRE VALUE OF FUNCTION      │
    │ STRENGTH                       │
    └───────────────────────────────┘
                │
                ▼
    ┌───────────────────────────────┐ ─── 1530
    │ CONTROL FUNCTION ENTITY FOR    │
    │ PREDETERMINED FUNCTION TIME AT │
    │ ACQUIRED FUNCTION STRENGTH     │
    └───────────────────────────────┘
                │
                ▼
  Yes   ◇─────────────────◇ ─── 1540
◄───────│  EXECUTION OF    │
        │ FUNCTION SHOULD BE│
        │    STOPPED?      │
        ◇─────────────────◇
                │
                No
                │
                ▼
        ◇─────────────────◇ ─── 1550
        │ VALUE OF FUNCTION │  Yes
        │ STRENGTH CAN BE FURTHER│────►
        │   ACQUIRED?      │
        ◇─────────────────◇
                │
                No
                │
                ▼
           ┌─────────┐
           │   END   │
           └─────────┘
```

# Fig. 16A

1600A

START

FUNCTION SHOULD
BE EXECUTED? —— 1610A

DETERMINE FUNCTION STRENGTH AND
FUNCTION TIME —— 1620A

ACTIVATE FUNCTION ENTITY
FOR DETERMINED FUNCTION TIME AT
DETERMINED FUNCTION STRENGTH —— 1630A

DETERMINE FUNCTION STOP TIME —— 1640A

WAIT FOR DETERMINED FUNCTION
STOP TIME —— 1650A

—— 1660A

EXECUTION OF
FUNCTION SHOULD BE
STOPPED?

No

Yes

END

# Fig. 16B

START

<u>1600B</u>

FUNCTION SHOULD BE EXECUTED? —— 1610B

No

Yes

ACQUIRE VALUES OF FUNCTION STRENGTH, FUNCTION TIME, AND FUNCTION STOP TIME —— 1620B

ACTIVATE FUNCTION ENTITY FOR ACQUIRED FUNCTION TIME AT ACQUIRED FUNCTION STRENGTH —— 1630B

WAIT FOR ACQUIRED FUNCTION STOP TIME —— 1640B

EXECUTION OF FUNCTION SHOULD BE STOPPED? —— 1650B

Yes

No

VALUES OF FUNCTION STRENGTH, FUNCTION TIME, AND FUNCTION STOP TIME CAN BE FURTHER ACQUIRED? —— 1660B

Yes

No

END

# Fig. 17

1700

```
            ┌─────────┐
            │  START  │
            └────┬────┘
                 │
                 ▼
  ┌─────────────────────────────────────┐
  │  DETECT MOTION OF FLAVOR INHALER     │──── 1710
  │         OR THE LIKE                  │
  └──────────────────┬──────────────────┘
                     │
                     ▼
  ┌─────────────────────────────────────┐
  │ CONVERT INPUT DATA INDICATING DETECTED │──── 1720
  │     MOTION TO FUNCTION DATA          │
  └──────────────────┬──────────────────┘
                     │
                     ▼
  ┌─────────────────────────────────────┐
  │  CONTROL FUNCTION OF FLAVOR INHALER  │──── 1730
  │    OR THE LIKE IN ACCORDANCE         │
  │       WITH FUNCTION DATA             │
  └──────────────────┬──────────────────┘
                     │
                     ▼
                ┌─────────┐
                │   END   │
                └─────────┘
```

## Fig. 18

1800

1802 — VIBRATOR

1803 — FUNCTION ENTITY

1804 — SENSOR

1806 — CONTROLLER

1808 — COMMUNICATOR

1810

CONTROLLER — 1816

CONVERTER — 1812

COMMUNICATOR — 1814

EP 4 413 884 A1

# Fig. 19

FLAVOR INHALER OR THE LIKE          EXTERNAL APPARATUS

1910 — DETECT MOTION OF FLAVOR INHALER OR THE LIKE

1912 — SEND INPUT DATA INDICATING DETECTED MOTION TO EXTERNAL APPARATUS

INPUT DATA →

RECEIVE INPUT DATA FROM FLAVOR INHALER OR THE LIKE — 1914

CONVERT INPUT DATA TO VIBRATION DATA FOR VIBRATING VIBRATOR OR FUNCTION DATA FOR CONTROLLING FUNCTION ENTITY — 1916

SEND VIBRATION DATA OR FUNCTION DATA TO FLAVOR INHALER OR THE LIKE — 1918

← VIBRATION DATA OR FUNCTION DATA

1920 — RECEIVE VIBRATION DATA OR FUNCTION DATA FROM EXTERNAL APPARATUS

1922 — VIBRATE VIBRATOR IN ACCORDANCE WITH VIBRATION DATA OR CONTROL FUNCTION ENTITY IN ACCORDANCE WITH FUNCTION DATA

EP 4 413 884 A1

# Fig. 20

2000

| |
|---|
| SENSORY STIMULATION ELEMENT — 2010 |
| SENSOR — 2020 |
| CONVERTER — 2030 |
| CONTROLLER — 2040 |

# Fig. 21

2100

START

DETECT MOTION OF FLAVOR INHALER
OR THE LIKE — 2110

WHEN INPUT DATA INDICATING DETECTED
MOTION IS BEING ACQUIRED, ACTIVATE AT LEAST
ONE SENSORY STIMULATION ELEMENT
CONFIGURED TO IMPART SENSORY
STIMULATION TO USER, TO FUNCTION — 2120

END

# Fig. 22

2200

```
                        ┌─────────┐
                        │  START  │
                        └─────────┘
                             │
        ┌────────────────────┼
        │                    ▼
        │           ╱─────────────────────╲              2210
        │          ╱      IS INPUT DATA     ╲
   No   │         ╱  INDICATING DETECTED MOTION ╲
        │◄───────◄   BEING CURRENTLY ACQUIRED   ►
        │         ╲        BY SENSOR?        ╱
        │          ╲──────────────────────╱
        │                   │
        │                  Yes
        │                   │
        │                   ▼
        │      ┌──────────────────────────────────┐
        │      │  ACQUIRE VALUE OF SENSORY STIMULATION │   2220
        │      │            STRENGTH               │
        │      └──────────────────────────────────┘
        │                   │
        │                   ▼
        │      ┌──────────────────────────────────┐
        │      │ ACTIVATE SENSORY STIMULATION ELEMENT FOR │
        │      │ PREDETERMINED SENSORY STIMULATION TIME │  2230
        │      │   AT SENSORY STIMULATION STRENGTH  │
        │      │  CORRESPONDING TO ACQUIRED VALUE   │
        │      └──────────────────────────────────┘
        │                   │
        │                   ▼
        │          ╱─────────────────────╲              2240
        │         ╱     IS OPERATION OF    ╲
   No   │        ╱     SENSOR STOPPED?      ╲
        └───────◄                            ►
                 ╲                          ╱
                  ╲────────────────────────╱
                            │
                           Yes
                            │
                            ▼
                        ┌─────────┐
                        │   END   │
                        └─────────┘
```

# Fig. 23

2300

START

IS INPUT DATA
INDICATING DETECTION MOTION
BEING CURRENTLY ACQUIRED
BY SENSOR? — 2310

DETERMINE SENSORY STIMULATION STRENGTH
AND SENSORY STIMULATION TIME — 2320

ACTIVATE SENSORY STIMULATION ELEMENT FOR
DETERMINED SENSORY STIMULATION TIME AT
DETERMINED SENSORY STIMULATION STRENGTH — 2330

DETERMINE SENSORY STIMULATION
STOP TIME — 2340

WAIT FOR DETERMINED SENSORY STIMULATION
STOP TIME — 2350

IS OPERATION OF
SENSOR STOPPED? — 2360

No

Yes

END

# Fig. 24

## Fig. 25

## Fig. 26

## Fig. 27

## Fig. 28

# Fig. 29

# Fig. 30

# Fig. 31

100A

3100

3102

3101

z'

y

x

x'

y'

z

3120

# Fig. 32

## INTERNATIONAL SEARCH REPORT

International application No.

**PCT/JP2022/037557**

### A. CLASSIFICATION OF SUBJECT MATTER

*A24F 40/50*(2020.01)i; *A24F 40/51*(2020.01)i; *G06F 3/01*(2006.01)i
FI:  A24F40/50; G06F3/01 510; A24F40/51

According to International Patent Classification (IPC) or to both national classification and IPC

### B. FIELDS SEARCHED

Minimum documentation searched (classification system followed by classification symbols)

A24F40/50; A24F40/51; G06F3/01

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 2021-058212 A (RAI STRATEGIC HOLDINGS INC) 15 April 2021 (2021-04-15) paragraphs [0068]-[0089], fig. 1-8 | 1-6, 11-15 |
| Y | | 7-10 |
| Y | JP 2020-126036 A (SEIKO EPSON CORP) 20 August 2020 (2020-08-20) paragraphs [0042]-[0043] | 7-10 |
| Y | JP 2017-192563 A (OMRON HEALTHCARE CO LTD) 26 October 2017 (2017-10-26) paragraphs [0022]-[0025] | 7-10 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **27 October 2022** | **15 November 2022** |

| Name and mailing address of the ISA/JP | Authorized officer |
|---|---|
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

INTERNATIONAL SEARCH REPORT
Information on patent family members

International application No.

**PCT/JP2022/037557**

| Patent document cited in search report | | | Publication date (day/month/year) | Patent family member(s) | | | Publication date (day/month/year) |
|---|---|---|---|---|---|---|---|
| JP | 2021-058212 | A | 15 April 2021 | US | 2016/0158782 | A1 | |
| | | | | paragraphs [0048]-[0069], fig. 1-8 | | | |
| | | | | CN | 107205484 | A | |
| | | | | CN | 113057383 | A | |
| | | | | EP | 3229623 | A1 | |
| | | | | EP | 3949768 | A1 | |
| | | | | ES | 2908405 | T3 | |
| | | | | HK | 1245591 | A1 | |
| | | | | JP | 2018-504095 | A | |
| | | | | PL | 3229623 | T3 | |
| | | | | WO | 2016/094225 | A1 | |
| JP | 2020-126036 | A | 20 August 2020 | US | 2020/0249253 | A1 | |
| | | | | paragraphs [0056]-[0057] | | | |
| | | | | CN | 111521843 | A | |
| JP | 2017-192563 | A | 26 October 2017 | (Family: none) | | | |

**EP 4 413 884 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2021058212 A
- JP 2017509339 W
- WO 2020008028 A
- WO 2020234053 A